# EUROPEAN PATENT APPLICATION

(11) **EP 2 107 120 A1**
(43) Date of publication of application: **07.10.2009**
(21) Application number: 08168266.8
(22) Date of filing: 24.07.2003
(51) Int. Cl.: C12Q 1/24, C12M 1/34

(54) **Capture and detection of microbes by membrane methods**

(30) Priority: 24.07.2002 US 398324 P; 24.07.2002 US 398203 P; 24.07.2002 US 398314 P; 24.07.2002 US 398235 P; 24.07.2002 US 398148 P
(62) Divisional of application: 03765999.2
(71) Applicant: Board of Regents, The University of Texas System, Austin, TX 78701 (US)
(72) Inventor: McDevitt, John T., Austin, TX 78735 (US); Floriano, Pierre, Austin, TX 78749 (US); Christodoulides, Nick J., Austin, TX 78704 (US)
(74) Representative: W.P. Thompson & Co.

(57) **Abstract**

Methods and systems for detecting the presence of analytes using a membrane based detection system are described. A fluid sample is passed through a membrane based detection system (100). Particulate analytes (e.g., microbes) are captured by the membrane (110). Detection and analysis techniques may be applied to determine the identity and quantity of the captured analytes.

## Description

### BACKGROUND OF THE INVENTION

### 1. Field of the Invention

The present invention relates to a method and device for the detection of analytes in a fluid. More particularly, the invention relates to the development of a sensor array system capable of discriminating mixtures of analytes, toxins, and/or bacteria in medical, food/beverage, and environmental solutions.

### 2. Brief Description of the Related Art

The development of smart sensors capable of discriminating different analytes, toxins, and bacteria has become increasingly important for clinical, environmental, health and safety, remote sensing, military, food/beverage and chemical processing applications. Many sensors capable of high sensitivity and high selectivity detection have been fashioned for single analyte detection. A smaller number of sensors been developed which display solution phase multi-analyte detection capabilities. One of the most commonly employed sensing techniques has exploited colloidal polymer microspheres for latex agglutination tests (LATs) in clinical analysis. Commercially available LATs for more than 60 analytes are used routinely for the detection of infectious diseases, illegal drugs, and early pregnancy tests. The vast majority of these types of sensors operate on the principle of agglutination of latex particles (polymer microspheres) which occurs when the antibody-derivatized microspheres become effectively "cross-linked" by a foreign antigen resulting in the attachment to, or the inability to pass through a filter. The dye-doped microspheres are then detected colorimetrically upon removal of the antigen carrying solution.

More recently, "taste chip" sensors have been employed that are capable of discriminating mixtures of analytes, toxins, and/or bacteria in medical, food/beverage, and environmental solutions. Certain sensors of this type are described in U.S. Application Ser. No. 10/072,800, METHOD AND APPARATUS FOR THE CONFINEMENT OF MATERIALS IN A MICROMACHINIED CHEMICAL SENSOR ARRAY, filed January 31, 2002 by McDevitt et al., which is incorporated by reference as if fully set forth herein. Disclosed therein are systems and methods for the analysis of a fluid containing one or more analytes. The taste chip array includes a sensor that has a plurality of chemically sensitive beads, formed in an ordered array, capable of simultaneously detecting many different kinds of analytes rapidly. An aspect of the system is that the array may be formed using a microfabrication process, thus allowing the system to be manufactured in an inexpensive manner.

Since concerns of bioterrorism attacks have become more pronounced, there has been increased interest in methods and systems for detecting microbes, particularly pathogens such as E. Coli O157:H7, B. anthracis/B. globigii, and Cryptosporidium, that may be used in chemical and biological attacks. Numerous high quality tests exist for the detection of microbes within research laboratory settings. However, these tests are generally expensive, time consuming, and require substantial laboratory resources. For many real-world applications in the health and safety, environmental, military, treaty verification and homeland defense areas, it is desirable to monitor numerous locations simultaneously, even locations where the majority of the time there will be no dangerous levels of microbes present.

Typical methods of detection, used for years by microbiologists, require the growth of single bacteria into bacterial colonies in different types of media, followed by a timely identification process involving morphological and biochemical tests. The classification of microorganisms through conventional microbiologal counting and enumeration methods involves the use of nucleic acid stains or cocktails of stains, which are capable of differentiating between gram-positive and gram negative bacteria, and between dead or living organisms. However, these procedures suffer from poor specificity and are not easily adapted to online rapid analysis. This series of steps, although often providing very accurate results repose on the expertise of highly trained personnel, and require lengthy and complicated analysis. Recent efforts have been directed towards developing approaches suitable for the entrapment or capture of bacteria, based on a combination of physical characteristics of the capturing medium and the affinity of the bacteria for a variety of chemical functionalities. Chemical associations with polymers, and with self-assembled monolayers (SAMs) have been used for bacterial capture applications. While rapid, these methods are non-specific, requiring completion of multi-step analysis for identification and quantification. A number of techniques, including PCR, involve the use of oligonucleotide probes and hybridization detection schemes. False positives, high cost, poor adaptability to multiplexing, and the need for trained personnel are major limitations of such approaches, despite their excellent specificity and sensitivity.

Great efforts have been made recently to decrease analysis time and improve sensitivity through the application of various techniques. Such techniques include polymerase chain reaction (PCR), electrochemical transduction, optical and microarray detection, flow-through immunofiltration, acoustic sensors, and flow cytometry.

Most commonly available assays for the detection of spores or bacteria involve the use of enzyme-linked immunosorbent assays (ELISA). While demonstrating high specificity, reproducibility, and capabilities of multiplexing through the use of specific antibodies, these methods generally require lengthy analysis times, and are not compatible with real-time analysis. Numerous methods have been adapted to combine the advantages of immunoassays and other analytical techniques in an effort to shorten analysis time, improve selectivity, and sensitivity. These techniques, however, rarely feature together the long list of attributes necessary for the creation of an "ideal sensor" as is demonstrated by the small number of commercially available sensing units.

It is therefore desirable that new methods and systems capable of discriminating microbes be developed for health and safety, environmental, homeland defense, military, medical/clinical diagnostic, food/beverage, and chemical processing applications. It is further desired that the methods and systems facilitate rapid screening of microbes to be used as a trigger for more specific and confirmatory testing. It is further desired that sensor arrays be developed that are tailored specifically to serve as efficient microbe collection media.

### SUMMARY OF THE INVENTION

Herein we describe systems and methods for the analysis of a fluid containing one or more analytes. The system may be used for either liquid or gaseous fluids. The system, in some embodiments, may generate patterns that are diagnostic for both individual analytes and mixtures of analytes. The system, in some embodiments, includes a plurality of chemically sensitive particles, formed in an ordered array, capable of simultaneously detecting many different kinds of analytes rapidly.

In an embodiment, a sensor array may contain one or more beads that contain macropores. Microbes such as bacteria, spores, and protozoa in a fluid may be captured in the macropores of the bead. In some embodiments, receptors, including, but not limited to, antibodies or semi-selective ligands such as lectins, may be coupled to a particle in an internal pore region of the bead to create a selective bead. In some embodiments, a visualization antibody may be introduced that may couple with the captured analyte to yield a colorimetric or fluorescence signature that can be recorded by the CCD detector. In some embodiments, a series of selective and semi-selective beads may be used in conjunction with the sensor array system described herein.

In some embodiments, a method for detecting microbes may include a multi-stage process wherein a fluid first undergoes a rapid screening and then, if warranted by the results of the screening stage, more specific and/or confirmatory testing. A sensor array including a macroporous bead may be used to conduct the specific and/or confirmatory testing.

Also described herein are methods for forming macroporous beads that may be used to detect a microbe. In an embodiment, a method for preparing a macroporous bead may include adding a dispersion of a hydrophilic emulsifier to an aqueous solution of a polymeric resin to form an oil-in-water emulsion, adding a solution of a hydrophobic emulsifier to the oil-in-water emulsion to form a water-in-oil emulsion; then cooling the water-in-oil emulsion to form a polymeric matrix in which a plurality of oil droplets are dispersed. The oil droplets may be washed out of the pores of the polymeric matrix to form a macroporous bead.

### BRIEF DESCRIPTION OF THE DRAWINGS

Features and advantages of the methods and apparatus of the present invention will be more fully appreciated by reference to the following detailed description of presently preferred but nonetheless illustrative embodiments in accordance with the present invention when taken in conjunction with the accompanying drawings in which:
FIG. 1 depicts an exploded view of a membrane based flow sensor;
FIG. 2 depicts an embodiment of a membrane based flow sensor disposed in a housing;
FIG. 3 depicts a schematic view of an analyte detection system in flow-through mode;
FIG. 4 depicts a schematic view of an analyte detection system in lateral flow mode;
FIG. 5 depicts a schematic view of an analyte detection system in back-flush mode;

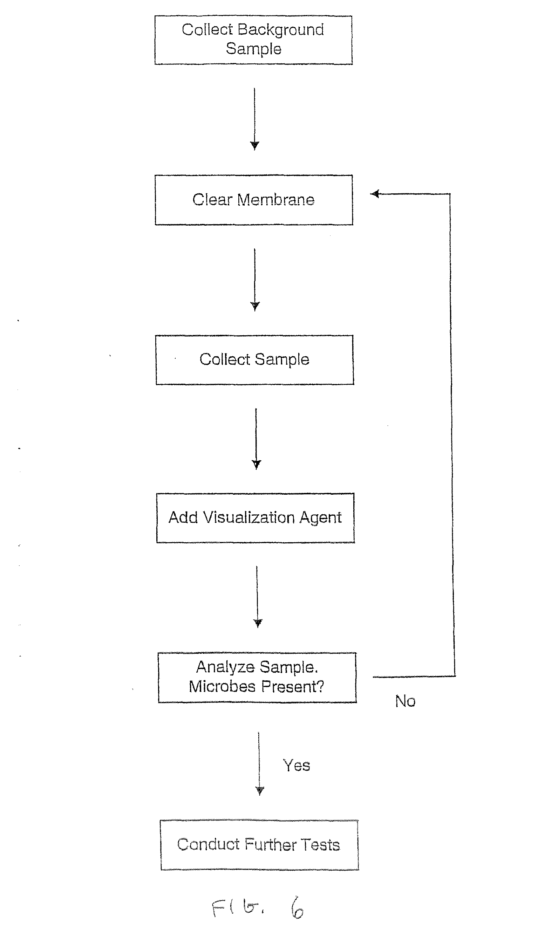

FIG. 6 depicts a flow chart of a method of collecting samples;
FIG. 7 depicts a flow chart of a method of collecting samples;
FIGS. 8A-8F depict a method of analysis of particles captured by a membrane;
FIG. 9 depicts a schematic diagram of a membrane based analyte detection system that includes a sensor array detection device;
FIG. 10 depicts porous particles;
FIGS. 11A-D depicts a schematic diagram of a bead optimization method; and
FIG. 12 depicts a schematic diagram of a flow cytometer.

### DETAILED DESCRIPTION OF PREFERRED EMBODIMENTS

Herein we describe a system and method for the analysis of a fluid containing one or more analytes. The system may be used for either liquid or gaseous fluids. The system, in some embodiments, may generate patterns that are diagnostic for both the individual analytes and mixtures of the analytes. The system in some embodiments, is made of a plurality of chemically sensitive particles, formed in an ordered array, capable of simultaneously detecting many different kinds of analytes rapidly. An aspect of the system is that the array may be formed using a microfabrication process, thus allowing the system to be manufactured in an inexpensive manner.

In an embodiment of a system for detecting analytes, the system, in some embodiments, includes a light source, a sensor array, and a detector. The sensor array, in some embodiments, is formed of a supporting member which is configured to hold a variety of chemically sensitive particles (herein referred to as "particles") in an ordered array. The particles are, in some embodiments, elements which will create a detectable signal in the presence of an analyte. The particles may produce optical (e.g., absorbance or reflectance) or fluorescence/phosphorescent signals upon exposure to an analyte. Examples of particles include, but are not limited to functionalized polymeric beads, agarous beads, dextrose beads, polyacrylamide beads, control pore glass beads, metal oxides particles (e.g., silicon dioxide (SiO₂) or aluminum oxides (Al₂O₃)), polymer thin films, metal quantum particles (e.g., silver, gold, platinum, etc.), and semiconductor quantum particles (e.g., Si, Ge, GaAs, etc.). A detector (e.g., a charge-coupled device "CCD") may be positioned below the sensor array to allow for the data acquisition. In another embodiment, the detector may be positioned above the sensor array to allow for data acquisition from reflectance of the light off of the particles.

Light originating from the light source may pass through the sensor array and out through the bottom side of the sensor array. Light modulated by the particles may pass through the sensor array and onto the proximally spaced detector. Evaluation of the optical changes may be completed by visual inspection or by use of a CCD detector by itself or in combination with an optical microscope. A microprocessor may be coupled to the CCD detector or the microscope. A fluid delivery system may be coupled to the supporting member of the sensor array. The fluid delivery system, in some embodiments, is configured to introduce samples into and out of the sensor array.

In an embodiment, the sensor array system includes an array of particles. The particles may include a receptor molecule coupled to a polymeric bead. The receptors, in some embodiments, are chosen for interacting with analytes. This interaction may take the form of a binding/association of the receptors with the analytes. The supporting member may be made of any material capable of supporting the particles, while allowing the passage of the appropriate wavelengths of light. The supporting member may include a plurality of cavities. The cavities may be formed such that at least one particle is substantially contained within the cavity. The sensor array may include a cover layer. A cover layer may be positioned at a distance above the surface of the sensor array, such that a channel is formed between the sensor array surface and the cover layer. The cover layer may be placed at a distance such that the cover layer inhibits dislodgement of the particles from the cavities in the sensor array, while allow fluid to enter the cavities through the channel formed between the sensor array and the cover layer. In some embodiments, the cavities may be configured to allow fluid to pass through the cavity during use, while the cavity is configured to retain the particle in the cavity as the fluid passes through the cavity.

In an embodiment, the optical detector may be integrated within the bottom of the supporting member, rather than using a separate detecting device. The optical detectors may be coupled to a microprocessor to allow evaluation of fluids without the use of separate detecting components. Additionally, a fluid delivery system may also be incorporated into the supporting member. Integration of detectors and a fluid delivery system into the supporting member may allow the formation of a compact and portable analyte sensing system.

A high sensitivity CCD array may be used to measure changes in optical characteristics which occur upon binding of the biological/chemical agents. The CCD arrays may be interfaced with filters, light sources, fluid delivery and micromachined particle receptacles, so as to create a functional sensor array. Data acquisition and handling may be performed with existing CCD technology. CCD detectors may be configured to measure white light, ultraviolet light or fluorescence. Other detectors such as photomultiplier tubes, charge induction devices, photo diodes, photodiode arrays, and microchannel members may also be used.

A particle, in some embodiments, possess both the ability to bind the analyte of interest and to create a modulated signal. The particle may include receptor molecules which posses the ability to bind the analyte of interest and to create a modulated signal. Alternatively, the particle may include receptor molecules and indicators. The receptor molecule may posses the ability to bind to an analyte of interest. Upon binding the analyte of interest, the receptor molecule may cause the indicator molecule to produce the modulated signal. The receptor molecules may be naturally occurring or synthetic receptors formed by rational design or combinatorial methods. Some examples of natural receptors include, but are not limited to, DNA, RNA, proteins, enzymes, oligopeptides, antigens, and antibodies. Either natural or synthetic receptors may be chosen for their ability to bind to the analyte molecules in a specific manner.

In one embodiment, a naturally occurring or synthetic receptor is bound to a polymeric bead in order to create the particle. The particle, in some embodiments, is capable of both binding the analyte(s) of interest and creating a detectable signal. In some embodiments, the particle will create an optical signal when bound to an analyte of interest.

A variety of natural and synthetic receptors may be used. The synthetic receptors may come from a variety of classes including, but not limited to, polynucleotides (e.g., aptamers), peptides (e.g., enzymes and antibodies), synthetic receptors, polymeric unnatural biopolymers (e.g., polythioureas, polyguanidiniums), and imprinted polymers. Polynucleotides are relatively small fragments of DNA which may be derived by sequentially building the DNA sequence. Peptides include natural peptides such as antibodies or enzymes or may be synthesized from amino acids. Unnatural biopolymers are chemical structure which are based on natural biopolymers, but which are built from unnatural linking units. For example, polythioureas and polyguanidiniums have a structure similar to peptides, but may be synthesized from diamines (i.e., compounds which include at least two amine functional groups) rather than amino acids. Synthetic receptors are designed organic or inorganic structures capable of binding various analytes.

In an embodiment, a large number of chemical/biological agents of interest to the military and civilian communities may be sensed readily by the described array sensors. Bacteria may also be detected using a similar system. To detect, sense, and identify intact bacteria, the cell surface of one bacteria may be differentiated from other bacteria, or genomic material may be detected using oligonucleic receptors. One method of accomplishing this differentiation is to target cell surface oligosaccharides (i.e., sugar residues). The use of synthetic receptors which are specific for oligosaccharides may be used to determine the presence of specific bacteria by analyzing for cell surface oligosaccharides.

In one embodiment, a receptor may be coupled to a polymeric resin. The receptor may undergo a chemical reaction in the presence of an analyte such that a signal is produced. Indicators may be coupled to the receptor or the polymeric bead. The chemical reaction of the analyte with the receptor may cause a change in the local microenvironment of the indicator to alter the spectroscopic properties of the indicator. This signal may be produced using a variety of signalling protocols. Such protocols may include absorbance, fluorescence resonance energy transfer, and/or fluorescence quenching. Receptor-analyte combination may include, but are not limited to, peptides-proteases, polynucleotides-nucleases, and oligosaccharides- oligosaccharide cleaving agents.

In one embodiment, a receptor and an indicator may be coupled to a polymeric resin. The receptor may undergo a conformational change in the presence of an analyte such that a change in the local microenvironment of the indicator occurs. This change may alter the spectroscopic properties of the indicator. The interaction of the receptor with the indicator may be produce a variety of different signals depending on the signalling protocol used. Such protocols may include absorbance, fluorescence resonance energy transfer, and/or fluorescence quenching.

In an embodiment, the sensor array system includes an array of particles. The particles may include a receptor molecule coupled to a polymeric bead. The receptors, in some embodiments, are chosen for interacting with analytes. This interaction may take the form of a binding/association of the receptors with the analytes. The supporting member may be made of any material capable of supporting the particles, while allowing the passage of the appropriate wavelengths of light. The supporting member may include a plurality of cavities. The cavities may be formed such that at least one particle is substantially contained within the cavity. A vacuum may be coupled to the cavities. The vacuum may be applied to the entire sensor array. Alternatively, a vacuum apparatus may be coupled to the cavities to provide a vacuum to the cavities. A vacuum apparatus is any device capable of creating a pressure differential to cause fluid movement. The vacuum apparatus may apply a pulling force to any fluids within the cavity. The vacuum apparatus may pull the fluid through the cavity. Examples of vacuum apparatuss include pre-sealed vacuum chamber, vacuum pumps, vacuum lines, or aspirator-type pumps.

Further details regarding these systems can be found in the following U.S. patent applications, all of which are incorporated herein by reference: U.S. Patent Application Serial No. 09/287,248 entitled "Fluid Based Analysis of Multiple Analytes by a Sensor Array"; U.S. Patent Application Serial No. 09/354,882 entitled "Sensor Arrays for the Measurement and Identification of Multiple Analytes in Solutions"; U.S. Patent Application Serial No. 09/616,355 entitled "Detection System Based on an Analyte Reactive Particle"; U.S. Patent Application Serial No. 09/616,482 entitled "General Signaling Protocols for Chemical Receptors in Immobilized Matrices"; U.S. Patent Application Serial No. 09/616,731 entitled "Method and Apparatus for the Delivery of Samples to a Chemical Sensor Array"; U.S. Patent Application Serial No. 09/775,342 entitled "Magnetic-Based Placement and Retention of Sensor Elements in a Sensor Array"; U.S. Patent Application Serial No. 09/775,340 entitled "Method and System for Collecting and Transmitting Chemical Information"; U.S. Patent Application Serial No. 09/775,344 entitled "System and Method for the Analysis of Bodily Fluids"; U.S. Patent Application Serial No. 09/775,353 entitled "Method of Preparing a Sensor Array"; U.S. Patent Application Serial No. 09/775,048 entitled "System for Transferring Fluid Samples Through A Sensor Array" (Published as U.S. Publication No.: 2002-0045272-A1); U.S. Patent Application Serial No. 09/775,343 entitled "Portable Sensor Array System"; and U.S. Patent Application Serial No. 10/072,800 entitled "Method and Apparatus for the Confinement of Materials in a Micromachined Chemical Sensor Array".

### Method of Testing for Microbes Using A Membrane System

In another embodiment, a membrane based flow sensor was prepared which is configured to accommodate the capture of microbes with a filter placed within the fluidics device. Microbes, whose size is larger than the pores of the filter, are captured in the flow cell assembly. The captured microbes may be analyzed directly or may be treated with visualization compounds.

A variety of microbes may be captured and analyzed using a membrane based flow sensor as described herein. As used herein, "microbe" refers to any microorganism, including but not limited to, a bacteria, spore, protozoan, yeast, virus, and algae. Some microbes that are of particular interested for detection include a variety of toxic bacteria. Examples of bacteria that may be detected using a membrane based flow sensor include, but are not limited to *Escherichia coli O157:H7, Cryptosporidium, Vibrio cholerae, Shigella, Legionnella, Lysteria, Bacillus globigii*, and *Bacillus anthracis* (anthrax). Viruses may also be detected using a membrane, including the HIV virus.

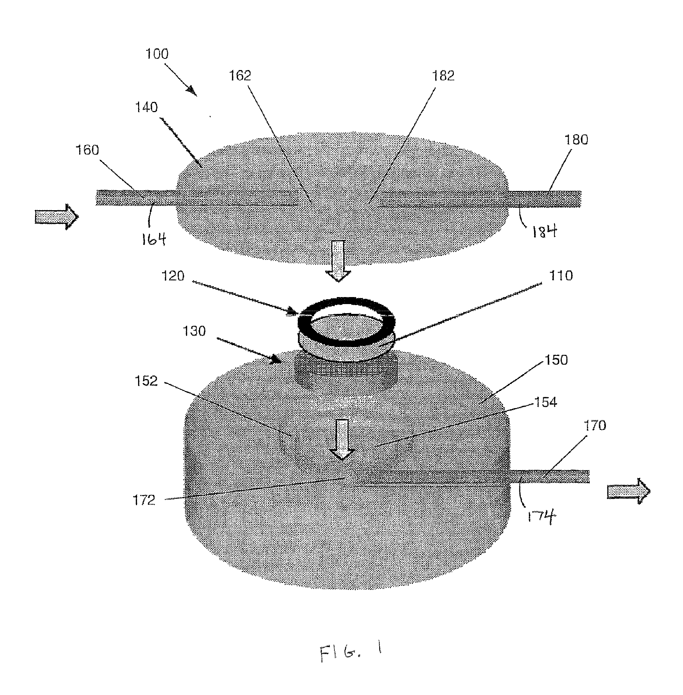

Shown in FIG. 1 is an exploded view of a membrane based flow sensor 100. Flow sensor 100 includes a membrane 110 that is sandwiched between at least two members 140 and 150. Members 140 and 150 are configured to allow fluid to flow to and through membrane 110. Members 140 and 150 are also configured to allow detection of analytes, after the analytes have been captured on membrane 110. A variety of different materials may be used for membrane 110, including, but not limited to, Nuclepore ® track-etched membranes, nitrocellulose, nylon, and cellulose acetate. Generally, the material used for membrane 110 should have resistance to non-specific binding of antibodies and stains used during the visualization and detection processes. Additionally, membrane 110 is composed of a material that is inert to a variety of reagents, buffers, and solvents. Membrane 110 may include a plurality of sub-micron pores that are fairly evenly distributed. The use of membranes having an even distribution of pores allows better control of fluid flow and control of the isolation of analytes.

Members 140 and 150 are composed of a material that is substantially transparent to wavelengths of light that are used to perform the analyte detection. For example, if the analyte detection method requires the use of ultraviolet light, member 140 should be composed of a material that is substantially transparent to ultraviolet light. Member 140 may be composed of any suitable material meeting the criteria of the detection method. A transparent material that may be used to form member 140 includes, but is not limited to, glass, quartz glass, and polymers such as acrylate polymers (e.g., polymethylmethacrylate). In some embodiments, both top member 140 and bottom member 150 are composed of transparent materials. The use of transparent materials for the top member and the bottom member allow detection to be performed through the membrane based flow sensor.

As shown in FIG. 1, membrane 110 is sandwiched between top member 140 and bottom member 150. Bottom member 150 and/or top member 140 may include indentations configured to hold a membrane. For example, in FIG. 1, bottom member 150 includes an indentation 152 that is configured to receive membrane 110, along with any other accompanying pieces that are used to support or seal membrane 110. Indentations or cavities may be etched into top member 140 and/or bottom member 150 using standard etching techniques.

Referring to FIG.1, bottom member 150 includes a first indentation 152, which is configured to receive a membrane support 130. Bottom member also includes a second indentation 154. Second indentation is configured such that membrane support 130 is inhibited from entering the second indentation. Second indentation may include a ridge disposed near the membrane support 130 such that membrane support 130 rests upon the ridge. Alternatively, as depicted in FIG. 1, second indentation may be to may have a size that is smaller than the size of membrane support 130. In either case, when assembled, membrane support 130 is inhibited from entering second indentation 154, thus creating a cavity under membrane support 130. Cavity 154 may be used to collect fluids that pass through the membrane support 130 prior to exiting the system.

Membrane support 130 is configured to provide support to membrane 110 during use. Membrane support 130 may be formed from a porous material that allows fluid to pass through the membrane support. The pores of membrane support 130 should have a size that allows fluid to pass through membrane support 130 at a speed that is equal to or greater than the speed that fluid passes through membrane 110. In one embodiment, pores of membrane support 130 are larger than pores in membrane 110. The pores, however, cannot be too large. One function of membrane support 130 is to provide support to membrane 110. Therefore, pores in membrane support 130 should be sufficiently small enough to inhibit sagging of membrane 110 during use. Membrane support 130 may be formed of a variety of materials including, but not limited to, polymeric materials, metals, and glass. In one embodiment, a polymeric material (e.g., celcon acrylic) may serve as a material for membrane support 130. Additionally, membrane support 130 helps to keep the membrane planar during use. Keeping the membrane planar simplifies detection of the analytes by allowing the capture and detection of the analytes on a single focal plane.

Membrane 110, as described above, may rest upon membrane support 130 when the membrane based flow sensor 100 is assembled. In some embodiments, a gasket 120, may be positioned on top of membrane 110. A gasket may be used to provide a fluid resistant seal between members 130 and 140 and membrane 110. Gasket may inhibit the leakage of fluid from the system during use.

Top member 140 may include a fluid inlet 160. Fluids for analysis may be introduced into device 100 via fluid inlet 160. Fluid inlet 160 may pass through a portion of top member 140. In some embodiments, a channel 162 may be formed in top member 140 such that tubing 164 may be inserted into channel 162. Channel 162 may turn near the center of the top member to deliver the fluids to an upper surface of membrane 110.

Bottom member 150 may include a fluid outlet 170. Fluids that are introduced into the device 100 via fluid inlet 160 pass through top member 140 and through membrane 110. The fluids are then collected in cavity 154. A fluid outlet 170 may pass through a portion of bottom member 150. In some embodiments, a channel 172 may be formed in bottom member 150 such that tubing 174 may be inserted into channel 172. Channel 172 may be positioned to receive fluids that are collected in cavity 154 during use.

Optionally, a washing fluid outlet 180 may be formed in top member 140. Washing fluid outlet 180 is configured to receive fluids that pass through or over membrane 110 during a washing operation. Washing fluid outlet 180 may pass through a portion of top member 140. In some embodiments, a channel 182 may be formed in top member 140 such that tubing 184 may be inserted into channel 182. Channel 182 may be positioned to receive fluids that are used to wash membrane 110 during use.

Membrane 110 is selected from a material capable of filtering the analytes of interest from a fluid stream. For examples, if microbes represent the analyte of interest, the filter should be capable of removing microbes from a fluid stream. A suitable membrane may include a plurality of pores that have a size significantly less than the size of the analyte of interest. For airborne toxic microbes (e.g., anthrax), the membrane may be configured to capture microbes that have a diameter of greater than about 1 µm. It is believed that microbes that have a diameter of less than about 1 µm are very difficult to generate in large quantities, and if the organisms are viable, environmental stresses tend to interfere with the action of the microbes due to the high surface area/mass ratio. Membranes may be formed from a variety of materials known in the art. In one embodiment, membrane 110 may be a track-etched Nuclepore^{™} polycarbonate membrane. A Nuclepore membrane is available from Whatman plc. Membrane 110 may be about 5-10 microns in thickness. Membrane 110 includes a plurality of pores. Pores may range from about 0.2 µm in diameter up to about 12 µm in diameter to capture potentially dangerous microbes.

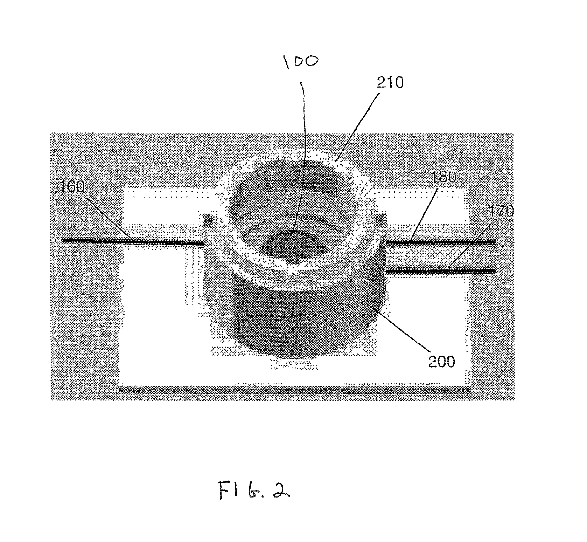

Fig. 2 depicts an embodiment of a membrane based flow sensor disposed in housing 200. Top member 140, gasket 120, membrane 110, membrane support 130, and bottom member 150 may be assembled and placed inside housing 200. Housing 200 may encompass membrane based fluid sensor. A cap 210 may be used to retain membrane based fluid sensor within housing 200. Cap 210 may include a window to allow viewing of membrane 110. When positioned within housing 200, fluid inlet 160, fluid outlet 170 and washing fluid outlet 180 extend from housing 200 to allow easy access to the membrane based fluid sensor 100.

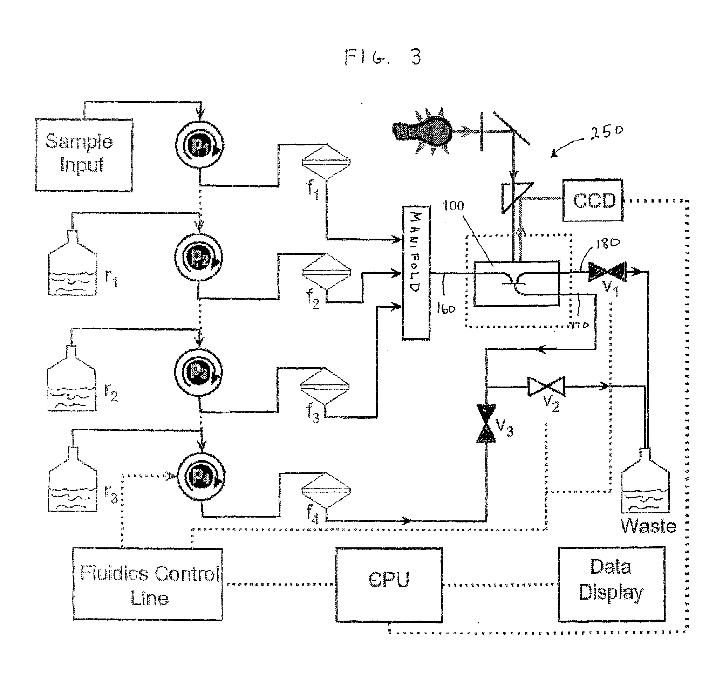

A schematic of a complete membrane based analysis system is shown in FIG. 3. Analysis system includes a plurality of pumps (p₁, p₂, p₃ and p₄). Pumps are configured to deliver samples (p₁), visualization reagents (p₂ and p₃) and membrane washing fluids (p₄) to the membrane based fluid sensor 100 during use. Reagents, washing fluids, and visualization agents are passed through pre-filters (f₁, f₂, f₃, and f₄) before the fluids are sent to membrane based fluid sensor 100. Pre-filters are used to screen out large particulate matter that may clog membrane 110. The nature and pore size of each pre-filter may be optimized in order to satisfy efficient capture of large dust particles or particulate matter aggregates while resisting clogging. Pre-filter f1 is configured to filter samples before the samples reach the membrane based fluid sensor 100. Pre-filter f1 is configured to allow the analyte of interest to pass through while inhibiting some of the particles that are not related to the analyte of interest. For example, spores, whose size is smaller than the pores of the pre-filter f₁, are passed through the pre-filter and captured in the membrane based fluid sensor 100. After passing through pre-filters f₁ - f₄, fluids are passed through a manifold. In some embodiments, membrane based fluid sensor 100 includes a single input line. The manifold couples the different fluid lines to the single input line of the membrane based fluid sensor 100.

After passing through the manifold, fluids are introduced into fluid inlet of the membrane based fluid sensor 100. At appropriate times, a detector 250 is used to determine if any analytes have been captured by the membrane based fluid sensor 100. As depicted in FIG. 3, a detector may be placed over a portion of membrane based fluid sensor 100 such that the detector may capture an image of the membrane. For example, detector may be placed such that images of the membrane may be taken through a window in the membrane based fluid sensor 100. Detector 250 may be used to acquire an image of the particulate matter captured on membrane 110. Image acquisition may include generating a "digital map" of the image. In an embodiment, detector 250 may include a high sensitivity CCD array. The CCD arrays may be interfaced with filters, light sources, fluid delivery, so as to create a functional sensor array. Data acquisition and handling may be performed with existing CCD technology. In some embodiments, the light is broken down into three-color components, red, green and blue. Evaluation of the optical changes may be completed by visual inspection (e.g., with a microscope) or by use of a microprocessor ("CPU") coupled to the detector. For fluorescence measurements, a filter may be placed between detector 250 and membrane 110 to remove the excitation wavelength. The microprocessor may also be used to control pumps and valves as depicted in FIG. 3.

The analyte detection system may be operated in different modes based on which valves are opened and closed. A configuration of a system in a "flow through" mode is depicted in FIG. 3. In this mode, fluid is passed from the manifold to the membrane based fluid sensor 100 to allow capture of analytes or the addition of development agents. Fluids for analysis may be introduced into membrane based fluid sensor 100 via fluid inlet 160. During a "flow through" operation, valve V₁ is placed in a closed position to inhibit the flow of fluid through wash fluid outlet 180. The fluids may, therefore, be forced to pass through membrane based fluid sensor 100 exit the sensor via fluid outlet 170. Valve V₂ is placed in an open position to allow the flow of fluid to the waste receptacle. Valve V₃ is placed in a closed position to inhibit the flow of fluid into the wash fluid supply line.

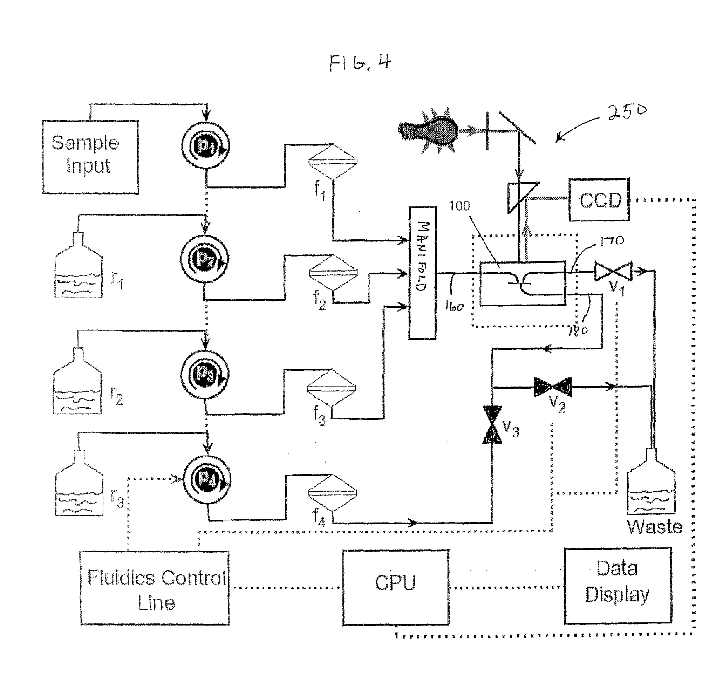

The analyte detection system may also be operated in a "lateral membrane wash" mode, as depicted in FIG. 4. In this mode, the membrane is cleared by the passage of a fluid across the collection surface of the membrane. This allows the membrane to be reused for subsequent testing. Fluids for washing the membrane may be introduced into sensor 100 via fluid inlet 160. During a "lateral membrane wash" operation, outlet valves V₂ and V₃ are placed in a closed position to inhibit the flow of fluid through fluid outlet 170. The closure of outlet valves V₂ and V₃ also inhibits the flow of fluid through the membrane of sensor 100. The fluids entering sensor 100 may, therefore, be forced to exit sensor 100 through washing fluid outlet 180. Valve V₂ is placed in an open position to allow the flow of fluid through washing fluid outlet 180 and into the waster receptacle. Since fluid is inhibited from flowing through the membrane, any analytes and other particles collected by the membrane may be "washed" from the membrane to allow further use.

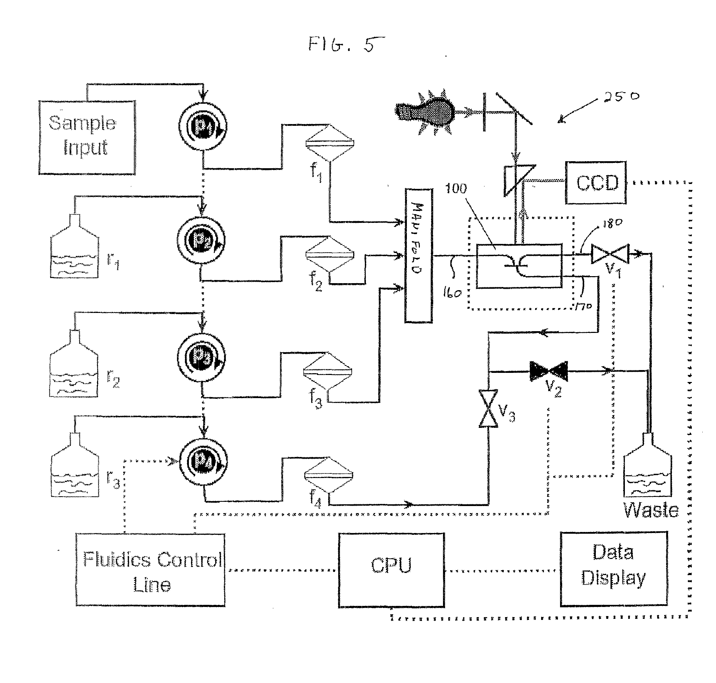

The analyte detection system may also be operated in a "backwash" mode, as depicted in FIG. 5. During a backwash operation, fluid outlet 170 is used to introduce a fluid into the analyte detection system, while wash fluid outlet 180 is used to allow the fluid to exit the device. This "reverse" flow of fluid through the cell allows the membrane to be cleared. In an embodiment, valves may be configured as depicted FIG. 5, with the washing fluid being introduced through fluid outlet 170. Specifically, valves V1 and V3 are open, while valve V2 is closed.

Either a lateral membrane wash or a back flush treatment may be used to clear analytes and other particles from a membrane. Both methods of clearing the membrane surface may be enhanced by the use of ultrasound or mechanical agitation. During use, analytes in the fluid sample are trapped by the membrane since the analytes are bigger than the openings in the membrane. The analytes tend to be randomly distributed across the membrane after use. Analytes that occupy positions on the membrane that are between the positions of pores may be harder to remove them analytes that are position on or proximate to a pore in the membrane. Analytes that occupy positions on the membrane that is between the positions of pores may be more difficult to remove, since the force of the backwash fluid may not contact the analytes. During backwash and lateral wash operations, removal of trapped analytes may be enhanced by the use of ultrasound of mechanical agitation. Both methods cause the analytes to move across the membrane surface, increasing the chances that the analyte will encounter a column of washing fluid passing through one of the pores.

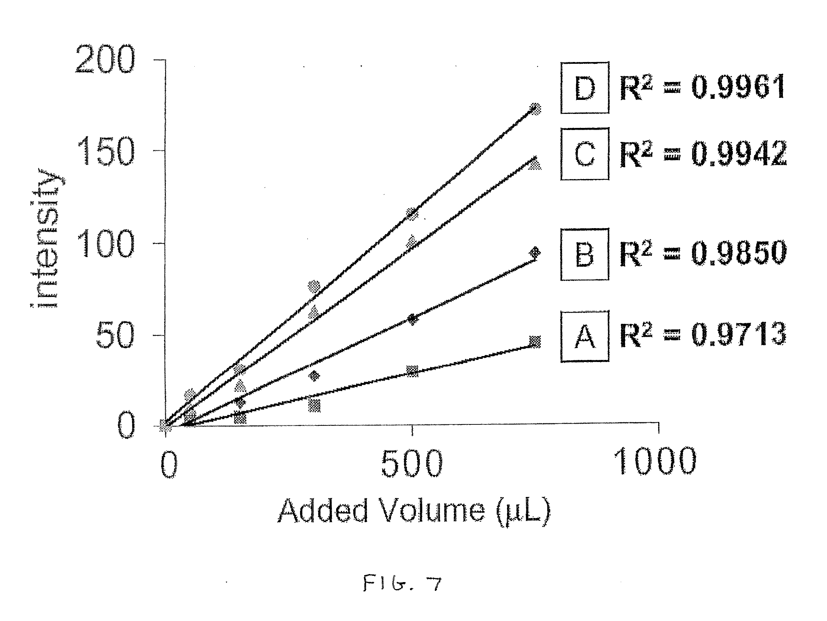

Analyte detection system may be used to determine the presence of analytes in a fluid system. One embodiment of a process for determining analytes in a fluid sample is depicted in the flow chart of FIG. 7. Prior to the analysis of any samples, a background sample may be collected and analyzed. Solid analytes are typically collected and stored in a liquid fluid. The liquid fluid that is used to prepare the samples, may be analyzed to determine if any analytes are present in the fluid. In one embodiment, a sample of the liquid fluid used to collect the solid analytes is introduced into an analyte detection device to determine the background "noise" contributed by the fluid. Any particles collected by the membrane during the background collection are viewed to determine the level of particulate matter in the liquid fluid. In some embodiments, particles collected by the membrane during the collection stage may be treated with a visualization agent to determine if any analytes are present in the liquid fluid. The information collected from the background check may be used during the analysis of collected samples to reduce false positive indications.

After collection of the background sample, the membrane may be cleared using either a back flush wash or a lateral wash, as described herein. After clearing the membrane, the system may be used to analyze samples for solid analytes (e.g., microbes). As used herein the term "microbes" refers to a variety of living organisms including bacteria, spores, viruses, and protozoa. As the collected sample is passed through the porous membrane, the porous membrane traps any particles that have a size that is greater than the size of the pores in the porous membrane. Collection of particles may be continued for a predetermined time, or until all of the collected sample has been passed through the membrane.

After collection, the particles collected by the membrane may be analyzed using a detector. In some embodiments, the detector may be a camera that will capture an image of the membrane. For example, a detector may be a CCD camera. Analysis of the particles captured by the membrane may be performed by analyzing the size and/or shape of the particles. By camparing the size and/or shape of the particles captured by the membrane to the size and shape of known particles the presence of a predetermined analyte may be indicated. Alternatively, microbe analytes will react to a variety of visualization agents (e.g., colored and fluorescent dyes). In one embodiment, the detection of microbe analytes may be aided by the staining of the microbe with a visualization agent. The visualization agent will induce a known color change or impart fluarescence to a microbe. In an embodiment, particles captured by the membrane are stained and the particles analyzed using an appropriate detector. The presence of particles that have the appropriate color and/or fluorescence may indicate the presence of the analyte being tested for. Typically non-microbe particles (e.g., dust) will not undergo the same color and/or fluorescent changes that microbes will when treated with the visualization agent. The visualization agent may include a "cocktail" mixture of semi-specific dyes, which may be designed to mark microbes of interest. Selection of the mixture may be based on the capacity of the dye chromophore to create an optical fingerprint that can be recognized by a detector and associated imaging software as being associated with specific pathogenic bacteria or spores, while at the same time distinguishing from the signal exhibited by dust and other background particulate matter.

The analysis of the particles may indicate that an analyte of interest is present in the sample. In this case, the particles may be flushed from the membrane and sent out of the system for further testing. Further testing may include techniques such as cultures or ELISA techniques that may allow more accurate determination of the specific analytes present. Alternatively, the particles may be sent to a sensor array, as described herein, for further testing. If no significant amounts of analytes are found on the membrane, the membrane may be washed and other samples analyzed.

In an embodiment, user-defined threshold criteria may be established to indicate a probability that one or more specific microbes are present on the membrane. The criteria may be based on one or more of a variety of characteristics of the image. In some embodiments, the criteria may be based on pixel or color fingerprints established in advance for specific microbes. The characteristics that may be used include, but are not limited to, the size, shape, or color of portions of matter on the image, the aggregate area represented by the matter, or the total fluorescent intensity of the matter. In an embodiment, the system may implement an automated counting procedure developed for one or more pathogenic and non-pathogenic bacteria.

In an embodiment, the membrane system may include a computer system (not shown). Computer system may include one or more software applications executable to to process a digital map of the image generated using detector. For example, a software application available on the computer system may be used to compare the test image to a pre-defined optical fingerprint. Alternatively, a software application available on computer system may be used to determine if a count exceeds a pre-defined threshold limit.

A detector may be used to acquire an image of the analytes and other particulate matter captured on a membrane. Microbes may collect on a membrane along with dust and other particulate matter and be captured in an image produced from a detector. The image acquired by the detector may be analyzed based on a pre-established criteria. A positive result may indicate the presence of a microbe. The test criteria may be based on a variety of characteristics of the image, including, but not limited to, the size, shape, aspect ratio, or color of a portion or portions of the image. Applying test criteria may allow microbes to be distinguished from dust and other particulate matter. During analysis, the flow of sample through from a fluid delivery system may be continued.

In some embodiments, a positive result may create a presumption that the fluid contains a particular analyte. If the image yields a positive result with respect to the test criteria, a sample of the fluid may be subjected to a confirmatory or specific testing. On the other hand, if the image yields a negative result with respect to the test criteria, membrane may be rinsed and the preceding method may be carried out for fluid from another sample.

During analyte testing a sample may be introduced into the analyte detection device. A trigger parameter may be measured to determine when to introduce the visualization agent into the analyte detection device. Measurement of the trigger parameter may be continuous or may be initiated by a user. Alternatively, the stain may be introduced into the analyte detection device immediately after the sample is introduced.

In one embodiment, the trigger parameter may be the time elapsed since initiation of introducing the fluid into an analyte detection device at a controlled flow rate. For example, the stain may be introduced 20 seconds after initiation of introducing the fluid sample into an analyte detection device at a flow rate of 1 milliliter per minute. In another embodiment, the trigger parameter may be the pressure drop across the membrane. The pressure drop across the membrane may be determined using a pressure transducer located on either side of the membrane.

In another embodiment, the trigger parameter may be the autofluorescence of analytes captured by the membrane. A detector may be switched on until a pre-defined level of signal from the autofluorescence of the analytes has been reached. In still another embodiment, filtering software may be used to create a data map of the autofluorescence of the matter on the membrane that excludes any pixels that contain color in a blue or red spectral range. The data map may be used to compute a value for particles that are autofluorescent only in the "pure green" portion of the visible spectrum.

In some embodiments, a presumptive positive result may be inferred if the trigger parameter exceeds a certain value without applying a stain. For example, a presumptive positive result may be inferred where the autofluorescence value is more than twice the value that would indicate application of a stain. In such a case, the application of a stain may be dispensed with and a confirmatory test may be conducted for the sample.

If the value of the trigger parameter is less than would indicate proceeding directly to the confirmatory test, but exceeds the value established to trigger the application of a stain, then a stain may be introduced into an analyte detection device.

Collecting a sample of a fluid may include gathering a sample from a solid, liquid, or gas. In some embodiments, the sample may be derived from collecting air from a target environment in an aerosol form, then converting aerosol into a hydrosol. For example, particles from 500 liters of an air sample may be collected deposited into about 0.5 milliliters of liquid. U.S. Patent No. 6,217,636 to McFarland, entitled "TRANSPIRATED WALL AEROSOL COLLECTION SYSTEM AND METHOD," which is incorporated herein by reference as if fully set forth herein, describes a system for collecting particulate matter from a gas flow into a liquid using a porous wall.

In one embodiment, a system as described above, may be used to determine the presence of anthrax spores or bacteria. Collection of air samples in a potentially contaminated area may be concentrated in a fluid sample using an airsol collector. The fluid sample may be passed through a membrane based detector system as described herein. The membrane based detection system may collect any particle collected by the airsol collector. The particles collected may be treated with a visualization agent that includes stains that are specific for anthrax bacteria. Such visualization agents are know to one of ordinary skill in the art. The presence of particles that exhibit the appropriate color/fluorescence may indicate that anthrax is presence. The indication of anthrax may be further determined by additional confirmation testing.

### Experimental

### Flow Cell

The flow cell assembly was created from a 3-piece stainless steel cell holder consisting of a base, a support and a screw-on cap. Two circular polymethylmethacrylate (PMMA) inserts house the nuclepore® membrane. These two PMMA inserts have been drilled along their edge and side to allow for passage of the fluid to and from the chip through stainless steel tubing (#304-H-19.5, Microgroup, Medway, MA). The tubes, which were fixed with epoxy glue in the drilled PMMA inserts had an outer diameter of 0.039" (19.5 gauge), and a 0.0255-0.0285" inner-diameter. The basic components of the flow cell are two disc-shaped PMMA "inserts". The bottom PMMA insert is modified in order to feature a drain and to contain a plastic screen disc (Celcon acrylic) that acts as a support for the filter. Each insert features a length of stainless steel tubing, which enters a hole in the side of the PMMA disk. The top insert also features an additional outlet which is used when regeneration of the filter is needed. Silicone tubing is snapped on the stainless steel tubing, and as such is readily compatible with a wide range of fluidic accessories (i.e., pumps, valves, etc.) and solvents. The flow cell was shown to be resistant to leaks and pressures generated by flow rates as high as 20 mL/min.

### Fluid Delivery, Optical Instrumentation and Software

The complete analysis system shown in FIGS. 3, 4, and 5 includes a fluidics system composed of four peristaltic pumps (p₁, p₂, p₃, and p₄), dedicated to the delivery of the analyte collected from the air, antibody, wash buffer to the flow cell, and clean-up off the flow cell in the regeneration mode. Its integrated software was used to assure fluid delivery to the chip, and accommodate wash cycles through the proper use of valves. The sample, antibody, PBS, and regeneration lines are also filtered (pre-filters f₁, f₂, f₃, and f₄) to screen out large particulate matter. Pre-filter f₁ is a nuclepare® filter with a pore size of 5 µm. Pre-filters f₂, f₃, f₄ are 0.4 µm nuclepore® filters. Spores which size is smaller than the pores of pre-filter f₁ are passed through the filter and captured in the analysis flow cell, positioned on the motorized stage of a modified compound BX2 Olympus microscope. The microscope is equipped with various objectives, optical filters, and a charged-coupled device (CCD) camera which operation can be automated.

A Mercury lamp was used as the light source. Fluorescence images shown in this report were obtained with a FTTC filter cube (fluoroisothiocyanate, 480 nm excitation, 505 long pass beam splitter dichroic mirror, and 535 ± 25 nm emission), and captured by a DVC 1312C (Digital Video Company, Austin, TX) charge-coupled device (CCD) mounted on the microscope and interfaced to Image Pro Plus 4.0 software (Media Cybernetics). Areas of interest of the images of the array for were selected in an automated fashion and used to extract numerical values of the red, green, and blue (RGB) pixel intensities.

### Reagents

Phosphate buffer saline (PBS), pH 7.4, was purchased from Pierce(# 28374, 0.008M Na₃PO₄, 0.14M NaCl, 0.01M KCl). The content of the pre-weighted pack was dissolved in 500 mL dI water. After complete dissolution, the buffer solution was filtered using a 60 mL disposable syringe (Becton Dickinson #309654) and a 0.2 mm pore size syringe filter (Whatman 25 mm, 0.2mm Polyethersulfone (PES) filters #6896-2502). Polyoxyethylene-Sorbitan Monolaurate (Tween-20) and Bovine Serum Albumine (BSA) were purchased from Sigma (# P-1379, and # A-0281). The anti-*bg* antibody was generously given to us by Tetracore, and tagged with a fluorophore. The naked Antibody was labeled according to the protocol described in the Alexa Fluor® 488 Protein labeling kit from Molecular Probes (# A-10235), and stored at 4°C. The fiinal concentration of the labeled anti-*bg* was 0.5 mg/mL. When prepared for the assay the antibody was diluted 50 times in a filtered (3 mL Disposable Syringes from Becton Dickinson # 309574; Syringe Filters from Pall Gelman 13mm, 0.2µm Acrodisc CR Polytetrafluoroethylene PTFE # 4423) solution of 1% BSA/PBS (0.01g of BSA per mL of PBS). The spore preparations were given to us by Edgewood / Dugway Proving Grounds. For their evaluation, the spores were memberd onto Petri dishes and grown with *Luria Bertani plating medium*. The medium is composed *of* Bacto Tryptone, Bacto Yeast Extract, Agar Technical purchased from Difco (# 211705, # 212750, # 281230 respectively), and NaCl purchased from EM (# SX0420-1). Distilled Water, de-ionized with a Barnstead Nanopure Column was autoclaved for 30min. at 121°C to sterilize it.

### Polymer Microsphere Solutions

The fluorescent polymer green microspheres were purchased from Duke Scientific Corporation (Palo Alto, CA). A bead stock solution was prepared by diluting several drops of the original bead solution in 500 mL of dI water. A bright line counting chamber, or hemacytometer (Hausser Scientific, Horsham, PA) was used to determine the exact concentration of this solution. The concentration of a solution is typically obtained from the average of several measurements following a well established protocol. The concentration of our stock solution was found to be 1,883,750 beads/mL ± 8539 or a relative standard deviation of 0.45 %. For the solutions used in Figure 3 and Figure 4, we used a 1 to 50 dilution of the stock solution, and added 50 µL, 100 µL, 150 µL, 200 µL, and 250 µL of that solution to the same flow cell, and captured images at different magnifications.

### Bg Spore solutions Preparation

A 1 mg/mL spore stock solution (A) was prepared in sterile water by suspending x mg of spores in x mL of sterile water. Solutions B, C, D, E, F, G, H and I with respective concentrations af 10e-1, 10e-2, 10e-3, 10e-4, 10e-5, 10e-6, 10e-7, and 10e-8 mg/mL were obtained by serial dilution of the stock solution A.

### Bg Spore solutions Characterization

The concentration of spores per mg of preparation was evaluated by growing colonies in a *Luria Bertani* culture media and expressed in Colonies Formation Unit (CFU) per mg of spore. 15g of Bacto Tryptone, 7.5g of Bacto Yeast Extract and 15g of NaCl were dissolved in 1.5L of sterile water. The pH was adjusted to 7.6 (Fisher Accumet pHmeter 620) using a 0.1N NaOH solution. 22.5g of Agar technical were then added to the preparation. The solution was heated in a microwave to allow completed dissolution and autoclaved for 30min. at 121 °C. After cooling, the media was poured in disposable sterile culture members (Fisher*brand* #08-757-12). The members were left until the media had totally solidified and then wrapped with parafilm for storage.

The number of CFU per mg of the Bg spore Preparation was evaluated as follows: 100 µL of solutions A to I were memberd in the culture media at 37°C for 24hrs. After incubation, colonies had grown enough to be counted. Only members with a statistical number of colonies (between 30 and 300) were used to calculate the number of CFU per mg of spore preparation. Solutions A to E had too numerous counts (TNC) and solution H and I had not enough counts (under 30). In addition, sterile water was also memberd as a negative control and gave 0 CFU. The average concentration was determined from the remaining members as 3 x 10⁸ CFU/mg of spore preparation.

### Assay optimization

The specificity of the Tetracore antibody for Bg spores was confirmed first by in-tube reactions and subsequent evaluation with fluorescence microscopy of stained spores on glass slides. The same antibody was then employed for the detection of Bg spores captured on the filter membrane of our system. A series of tests were performed in order to identify those conditions resulting in the highest signal to noise ratio for this on-line assay. Parameters evaluated included: a) the effect of pre-treating the system's tubing and filter membrane with BSA (i.e. blocking of non-specific binding sites for the detecting antibody), b) varying the rate (i.e. flow rate) of antibody introduction to the flow cell, c) varying the antibody concentration, d) varying the incubation time of the antibody with *Bg* spores, e) identifying the optimal exposure time for image capture, and f) comparison of uni-directional mode of antibody flow to the cell versus re-circulation. Our studies revealed that blocking the system's tubing and the flow cell's filter membrane with BSA offered no significant advantage for the assay in terms of reducing the non-specific signal. Nonetheless, we found that when 1% BSA was included in the antibody solution, the *Bg*-specific signal was enhanced, resulting in a higher signal to noise ratio and, therefore, a more sensitive assay. An incubation time of *Bg* spores for five minutes with 1.5 mL of *Bg*-specific antibody at 10 µg/mL, which was introduced in the flow cell in uni-directional mode (i.e. in to flow cell and out to waste) at 0.3 mL/min were identified as the optimal conditions for the assay.

Our studies also showed that re-circulation of the antibody did not offer any advantage in terms of shortening the assay time or decreasing its detection limit. Even though such an approach could potentially reduce the amount of antibody utilized in the assay, we decided against it because prolonged re-circulation of the antibody was associated with its precipitation. As expected, precipitated antibody could be captured by the membrane and thus result in an increase of the non-specific signal. On the contrary, there was very little precipitation of the detecting antibody when delivered in uni-directional mode. We equipped the system with a 0.4 µm pre-filter, which prevented any precipitated antibody from reaching the analysis flow cell. This approach resulted in a much cleaner assay.

Finally, we determined that the appropriate exposure time for capturing the final images for this assay was 184 ms. This exposure time was such that it produced the strongest *Bg*-specific signal and the weakest background, non-specific signal resulting from contaminants such as dust, irrelevant unstained bacteria and fluorescent paper fibers that could potentially be found in the system.

### Dose response curve

To establish the standard curve, the spore solutions were prepared in a similar fashion as described previously with PBS instead of sterile water. Briefly, a 1mg/mL (or 3 x 10⁸ CFU/mL) spore stock solution A was prepared by suspending 1 mg of spores in 1 mL of PBS. Solutions B, C, D, E, F and G were obtained from stock solution A by serial dilution, resulting in concentrations of 3 x 10⁸, 3 x 10⁷, 3 x 10⁶, 3 x 10⁵, 3 x 10⁴, 3 x 10³, 3 x 10² CFU/mL respectively for solutions A, B, C, D, E, F, and G. These concentrations cover the range from 1 ng/mL to 1 mg/mL. For each solution, an assay was conducted through execution of the following steps. The solution is introduced through pump 1 for 60 s at a flow rate of 1mL/mn, and followed by a 60 s PBS wash through pump 2 with the same flow rate. The antibody is then slowly (0.3 mL/mn) passed through pump 3 to the flow cell. A final 90 s wash ensures the removal of any unbound or non-specifically attached antibody. The background signal was evaluated through five independent measurements of the signal obtained from the passage of antibody in five different spore-free flow cells. The limit of detection was chosen as 3 times the standard deviation obtained from the average of these five measurements. The calibration curve was built from the measurement of four different spore solutions accounting for 900, 3000, 9000, and 30000 spores. A fluorescent micrograph of the signal remaining after the final wash was recorded and the signal expressed as the density of green intensity per pixel. The average green density per pixel was plotted as a function of spore count determining a limit of detection of 900 spores.

### Electron Microscopy

Correlative light and electron microscopy was accomplished by placing a 5 µL aliquot of antibody-stained spores on a Formvar-coated TEM grid (Maxtaform H2 finder grids, Ted Pella, Inc). Due to the thick walls of the spores, it was possible to avoid more complex dehydration regimens and simply allow the spore suspension to air dry. After a suitable area was located and photographed with fluorescence microscopy, the grid was placed in a Philips 420 TEM and the same grid square was photographed. The grid was then affixed to an aluminum stub with carbon tape and sputter-coated with gold palladium. Using a Leo 1530 SEM, images were captured from the area of interest.

### Bead tests

In order to determine the functionality as well as the analytical validity of our system, we tested our integrated system with 2.3 µm and 1 µm fluorescent polymer microspheres (purchased from Duke Scientific Corporation). The size of these particles was chosen to best simulate populations of spores and bacteria. The calibration curves displaying the average density per pixel as a function of added volume are shown in Figure 7. Examination of these graphs reveals that the linearity of the detected response is not affected by the magnification. However, as expected, the slape of the regression lines increases with increasing magnification as the signal from the beads is brighter at high magnification. Many factors, such as the size and brightness of the bacteria or spores, the total area of the membrane exposed to the analyte, the field of view, dictate the experimental parameters to be used. Because they are very homogeneous in size and intensity, polymeric beads represent an ideal calibrator and simulant for spores. However, the actual size of spores is slightly smaller than that of the beads that were used, and the signal produced from a single spore-antibody-fluorophore complex is much less intense than that of the microspheres. Additionally, fluidics concerns prevent us from using too small a filter area, because the internal pressure is greatly raised as the fluid is forced through a dramatically reduced number of pores. Because the magnification does not change the linearity of the calibration curves as shown in Figure 7, and in order to accommodate a sustained flow through the flow cell, an objective of 5x, for a total magnification of 100x was chosen for the assay.

### Spores and Bacteria

To illustrate the capabilities of our detection system, we targeted *Bacillus globigii* (*Bg*), a commonly used non-pathogenic simulant for *Bacillus anthracis* (*Ba*). An immunoassay was created, based on the capture of *Bg* spores and their interaction with a *Bg*-specific antibody resulting in the formation of an immuno-complex. The effect of possible interferences in the assay was also tested with a variety of species such as yeast, talc powder, and other species of Bacillus as will be discussed later in this report. In Figure 5 is shown a fluorescent micrograph of *Bg* spores stained with an Alexa® 488-labeled *anti-Bacillus globigii* antibody. The schematic of the immuno-complex is shown in the inset. In order to demonstrate the specificity of the interaction of the anti-Bg antibody with the *Bg* spores, we conducted some correlation studies between the fluorescence micrographs and the images obtained from transmission electron microscopy (TEM) and scanning electron microscopy (SEM). An aliquot of immuno-labeled Bg was placed on a Formvar-coated TEM finder grid, and epifluorescence micrographs were obtained at various magnifications. The grids were then imaged with transmission electron microscopy (TEM), after which they were coated with gold palladium and imaged with scanning electron microscopy (SEM). As illustrated by the correspondence of the fluorescence signal with the position of the spores, the finder grid made it possible to unequivocally locate the same area in each instrument, clearly indicating that the fluorescence signal arises from the Alexa® 488-tagged antibody that is specifically binding to the *Bg* spores. Fluorescence micrographs obtained at a total magnification of ≈ 400x are shown in order to better represent this correlation. However, the correlation of the fluorescence signal from spores with TEM or SEM micrographs is also established with magnification as low as = 100x.

To determine the limit of detection of our system, we conducted a dose-dependence study. Solutions of spores were prepared by serial dilution of a stock spore solution, presuming that 1 mg of dry spores per mL yields 10⁸ spores per mL. Following the flow cell experiments, aliquots of the spore solutions were memberd to determine the exact spore concentration in terms of colony forming units per mL (CFU). The background was determined as the signal obtained after passage of the antibody through a blank filter and subsequent rinsing with PBS. In order to assess the limit of detection, the standard deviation was calculated from the average of 5 such measurements of the background. The limit of detection was established to be 900 spores.

### Considerations on dust and contaminants

As the internal volume of the flow cell is very small, it is necessary to flush out all contaminants in order to avoid clogging of the membrane filter. Of particular importance for these studies is the control of dust, commonly and abundantly found in the postal environment. SEM studies (not shown) have demonstrated that the dust produced through transport, manipulation, and processing of postal mail, contains fibers, debris, and various kinds of bacteria. Most significantly, dust contains a large number of particles with a wide size distribution encompassing the size range of the biological agents of interest. Furthermore, many of the dust components exhibit autofluorescence, due to the use of fluorescent brighteners and inks in the paper and document industries. Many of the trigger systems currently used in military type detectors repose on size selection principles such as Aerodynamic Particle Sizing (APS) or Flow Cytometry (FC), and for the reasons exposed previously, do not appear as the ideal trigger systems. Our system was tested in a blind study against triggering by yeast, talc, and powdered detergents. The rate of success was 100% as no false positive was generated. Another major potential problem arising from accumulation of dust in our system is clogging of the nuclepore® filter. We have conducted studies which showed that failure of the flow cell operation occurs only after 60 mg of dust are passed through, building a pressure greater than 60 psi, corresponding to 400 hours of postal operation, assuming that the concentration of dust reaching the flow cell is an average 6.2 µg/L. However, this result is widely dependent on the efficiency of the aerosol system and it is based on the assumption that the aerosol collection system has a built-in capability of discarding at least 95% of dust particles of 10 µm or higher. In these conditions, even though the accumulation of dust in the flow cell is inevitable in the long run, the device still exhibits a lifetime well above that desired for military applications. Additionally, we have shown that it is possible to regenerate the flow cell and extend its lifetime by flushing out up to 99 % of the dust, spores, or debris accumulated on the filter. This function can easily be implemented through the use of an additional outlet within the top insert of the flow cell, and implementation of an automated flush protocol. A combined method of sonication, backflow, and lateral flow is used to eliminate unwanted material from the membrane. This allows for extended operation of the detection system without the attention of a technician. The removal of spore-sized (0.93 µm) fluorescent polymer microspheres from the membrane surface during five consecutive trials was performed. Surface plots in column i represents the initial loading of the membrane in the flow cell. Efficiencies of 95%, 98%, 99%, 99%, 99% is reached, respectively, for the five trials.

### Pixel Analysis Methods for Detection of Microbes

In some embodiments, pixel analysis methods may be used in the analysis of an image of a fluid or captured matter. For example, pixel analysis may be used to discriminate microbes from dust and other particulate matter captured on a membrane. Pixel analysis may include analyzing characteristics of an image to determine whether a microbe is present in the imaged fluid.

Pixel analysis may be based on characteristics including, but not limited to, the size, shape, color, and intensity ratios of an image or portions of an image. As an example, the total area that emits light in an image may be used to conduct analysis. As another example, the green fluorescent intensity of an image may be used to conduct analysis. In an embodiment, an "optical fingerprint" for a specific microbe or set of microbes may be established for use in pixel analysis. In some embodiments, pixel analysis may be based on ratios between values, such as an aspect ratio of an element of matter captured on an image. In other embodiments, pixel analysis may be based on threshold values.

During use, a visualization agent may cause different particles to emit different wavelengths of light depending on the nature of the particle. When the particles are analyzed with a camera, a user may be able to determine if a particular analyte is present based on the color of the particle. For example, a green particle may indicate the presence of an analyte of interest. Any other colored particles may not be of interest to a user. While a person may be able to discern between colors, it is desirable for a computer system to also be able to discern different colors from a membrane sample. Many detectors can only discern specific colors when analyzing an image. For example, many CCD detectors can only discern red, blue and green colors. Thus, a CCD detector may not be able to discern the difference between a particle that emits both blue and green light and a particle that just emits green light, although the color difference may be apparent to a person using the system. To overcome this problem a method of subtracting out particles having the "wrong" color may be used.

In some embodiments, pixels of an image that do not fall within a color range specified by a user may be discarded from the image. In one embodiment, a fluid may be stained to cause a microbe of interest to emit light in only the green portion of the visible spectrum. By contrast, dust and other particles contained in the fluid may emit light in combinations of green, blue, and red portions of the visible spectrum in the presence of the stain. To isolate the portion of the image that represents only the microbe of interest, binary masks may be created to eliminate light emissions caused by non-microbial matter from the image.

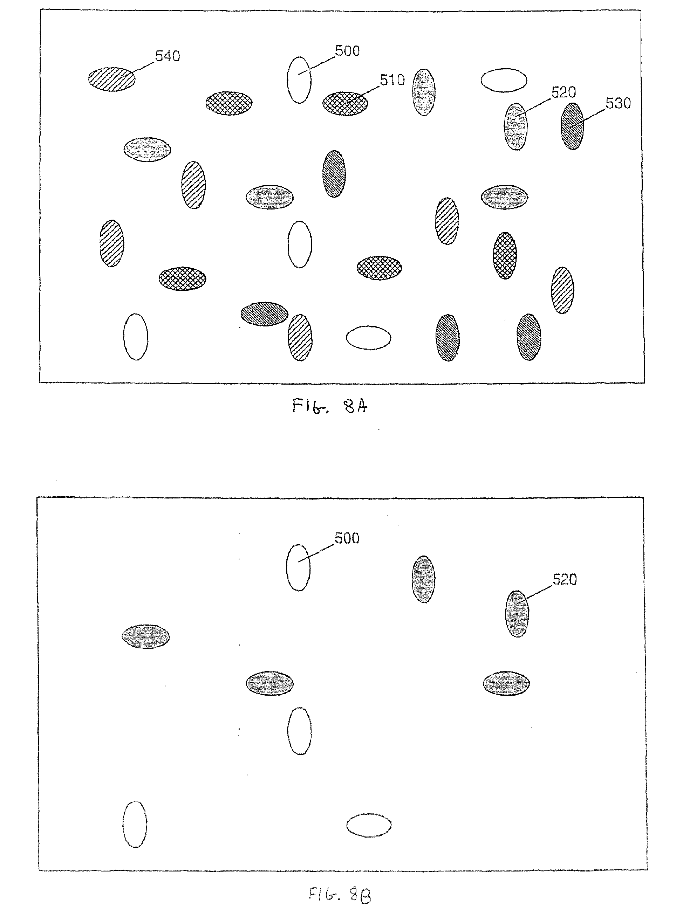

Such a method is depicted in FIGS 8A-F. FIG. 8A shows an image of all particles captured by a membrane. For purposes of this example, particles 500, having the no fill pattern, exhibit a green color; particles having a fill pattern identical to the fill pattern of particle 510 have a red color; particles having the a fill pattern identical to the fill pattern of particle 520 have both green and blue light absorption; particles having a fill pattern identical to the fill pattern of particle 530 have both red and blue light absorption; and particles having a fill pattern identical to the fill pattern of particle 540 have a blue color. It should be understood that these color assignments are for illustrative purposes only. In the current example, the goal of the analysis is to find all of the green particles.

One method of finding the green particles is to use a filter that will allow only particles that are green are shown. FIG. 8B depict the particles that would remain if such a filter is used. All of the particles shown in FIG. 8B have a green light absorption, however, not all of the particles that are depicted in FIG. 8B would exhibit a green color only. Particles 520 absorb both green and blue light. Since the detector can't differentiate between the two types of particles, a false positive may result.

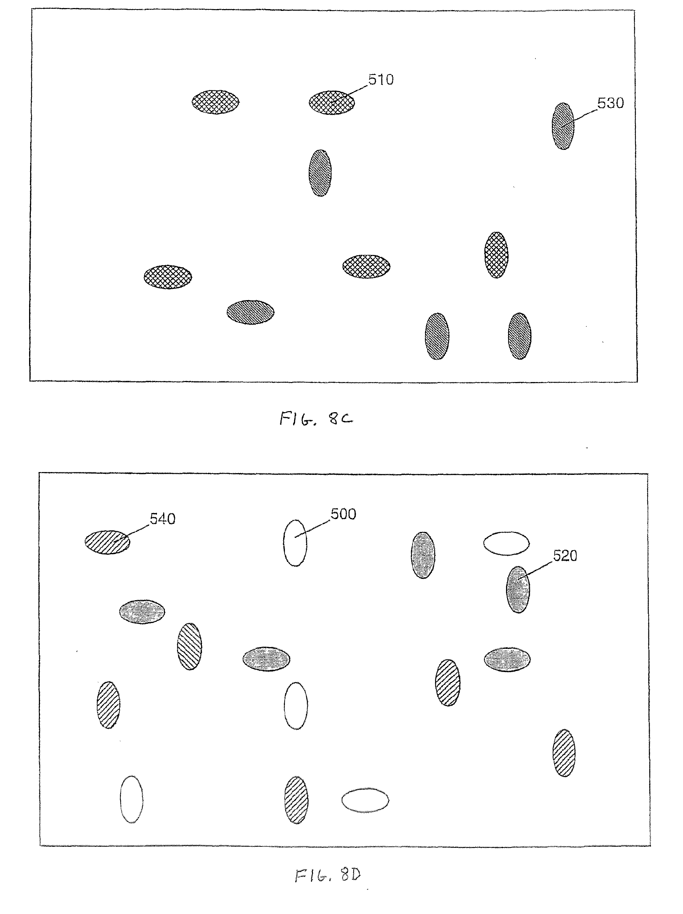

To compensate for this phenomena, images of particles that absorb blue and red are also analyzed using appropriate filters. By creating masks of which particles exhibit blue and red absorption, a process of elimination may be used to determine how many green particles are present. In an embodiment, an image is then captured of only the particles that exhibit color in the red portion of the spectrum (See FIG. 8C). The image of "red" particles is used to create a mask that may be compared to the full spectrum view of the particles. Since the analytes of interest only exhibit color in the green portion of the spectrum, any particle with color in the red portion of the spectrum may be removed from the original image. FIG. 8D shows the original image but with the particles that appear in the red portion of the spectrum subtracted from the image. The remaining particles are potential particles that may be the analyte of interest.

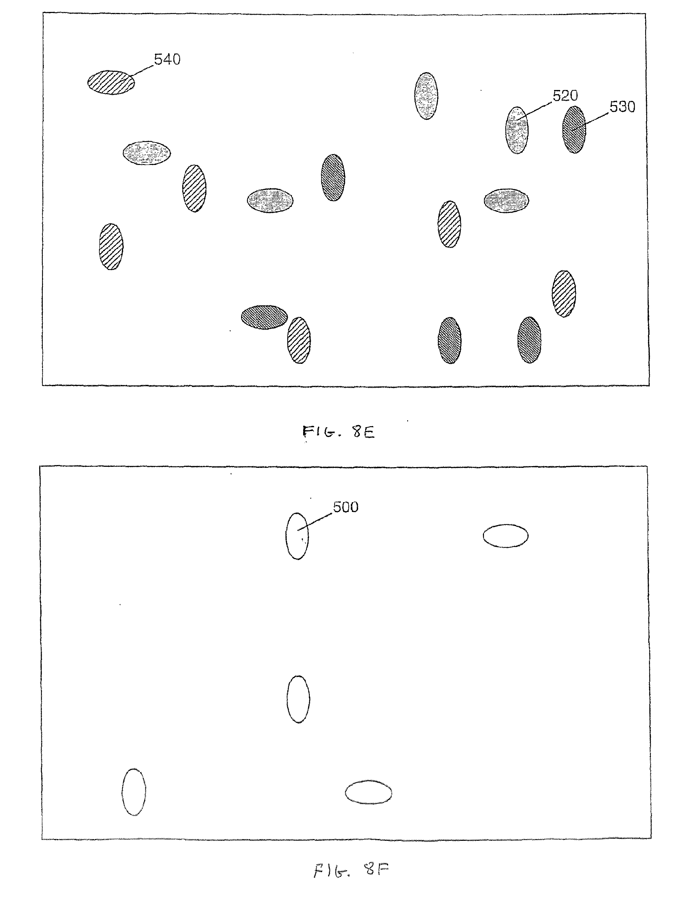

In a second iteration FIG. 8E shows a binary mask that may be used to mask any pixels that include a blue component. An image is captured of only the particles that exhibit color in the blue portion of the spectrum (See FIG. 8E). The image of "blue" particles is used to create a mask that may be compared to the full spectrum view of the particles. Since the analytes of interest only exhibit color in the green portion of the spectrum, any particle with color in the blue portion of the spectrum may be removed from the original image. FIG. 8F shows the original image but with the red binary mask and blue binary mask applied so that pixels including a red or blue component are excluded. The particles that remain in the image are thus particles that only exhibit a green color. Thus, the method may be used to produce an image that includes only "pure green" such an may be analyzed to detect the presence of a microbe by eliminating particles that are not relevant. It should be understood that while the above recited example is directed to determining the presence of green particles it should be understood that the process can be modified to determine blue particles only, red particles only, or particles that exhibit combinations of colors (e.g., red and blue, red and green, blue and green, or red, blue and green).

In some embodiments, pixel analysis may be used in combination with the membrane method for detecting a microbe described herein. Pixel analysis may be conducted either before or after the application of a stain. In an embodiment, pixel analysis may be used to determine when to apply a stain.

After an analyte of interest is detected using a membrane based detection device further testing may be performed to identify the analyte. In one example, the particles captured by the membrane may be transferred to a sensor array as described in any of the following U.S. Patent Applications: U.S. Patent Application Serial No. 09/287,248 entitled "Fluid Based Analysis of Multiple Analytes by a Sensor Array"; U.S. Patent Application Serial No. 09/354,882 entitled "Sensor Arrays for the Measurement and Identification of Multiple Analytes in Solutions"; U.S. Patent Application Serial No. 09/616,355 entitled "Detection System Based on an Analyte Reactive Particle"; U.S. Patent Application Serial No. 09/616,482 entitled "General Signaling Protocols for Chemical Receptors in Immobilized Matrices"; U.S. Patent Application Serial No. 09/616,731 entitled "Method and Apparatus for the Delivery of Samples to a Chemical Sensor Array"; U.S. Patent Application Serial No. 09/775,342 entitled "Magnetic-Based Placement and Retention of Sensor Elements in a Sensor Array"; U.S. Patent Application Serial No. 09/775,340 entitled "Method and System for Collecting and Transmitting Chemical Information"; U.S. Patent Application Serial No. 09/775,344 entitled "System and Method for the Analysis of Bodily Fluids"; U.S. Patent Application Serial No. 09/775,353 entitled "Method of Preparing a Sensor Array"; U.S. Patent Application Serial No. 09/775,048 entitled "System for Transferring Fluid Samples Through A Sensor Array" (Published as U.S. Publication No.: 2002-0045272-A1); U.S. Patent Application Serial No. 09/775,343 entitled "Portable Sensor Array System"; and U.S. Patent Application Serial No. 10/072,800 entitled "Method and Apparatus for the Confinement of Materials in a Micromachined Chemical Sensor Array".

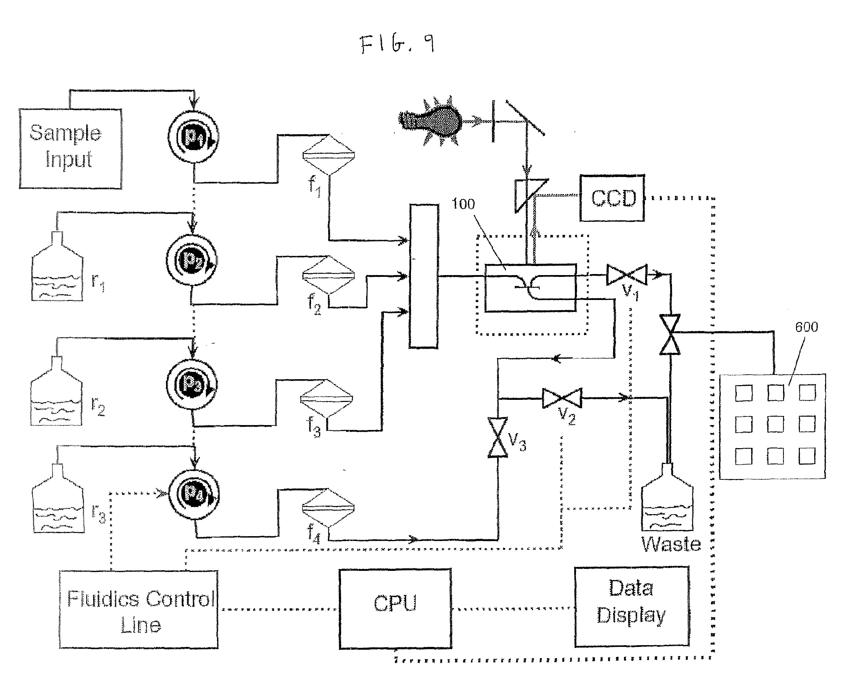

FIG. 9 depicts a system in which a particle sensor array detector 600 is coupled to a membrane analyte detection device 100. Membrane based analyte detection device may be part of an analyte detection system as previously described. After a sample is passed through a membrane, the particles collected by the membrane may be subjected to an additional test to further identify the analytes. In one embodiment, the analytes may be washed from the surface o the membrane and transferred to a sensor based analyte detection system, as described in any of the previously referenced patent applications. The analytes extracted from the sample may react with beads that are placed in a sensor array. The reaction of the analytes with the sensor array beads may allow confirmation (or further identification) of the analytes. Methods of detecting microbes using a sensor array system are described in further detail in the above-referenced patent applications.

Many microbes may not react with a bead of a sensor array. Large microbes may be unable to make proper contact with the bead and therefore are not detected by the bead. In one embodiment, the microbes are subjected to a treatment that allows better detection by a bead based detection system. In one embodiment, the particles may be subjected to lysis conditions. Lysis of microbes will cause the disintegration or dissolution of the microbe. For bacteria, lysis may be induced by treatment with an alkali base or by use of enzymes. Lysis of the bacteria allows portions of the material contained by the bacteria to be released and analyzed. Typically, either proteins or nucleic acids released from the bacteria may be analyzed.

Microbes such as bacteria, spores, and protozoa in a fluid may be captured in the macropores of the beads. In some embodiments, receptors, including, but not limited to, elective antibodies or semi-selective ligands such as lectins, may be coupled to a particle in an internal pore region of the particle to create a selective bead. Suitable receptors may be selected using the methods described herein. In some embodiments, a visualization antibody may be introduced that may coupled with the captured analyte. The visual antibody may yield a colorimetric or fluorescence signature that can be recorded by the CCD detector. In some embodiments, a series of selective and semi-selective beads may be used in conjunction with the sensor array system described herein.

In an embodiment, an agent that is known to bind or interact with a microbe may be introduced into a fluid prior to the time that the microbes are placed in proximity with a sensor array. Such agents may have characteristics that facilitate capture of a microbe by a particle in the sensor array.

### Macroporous particles

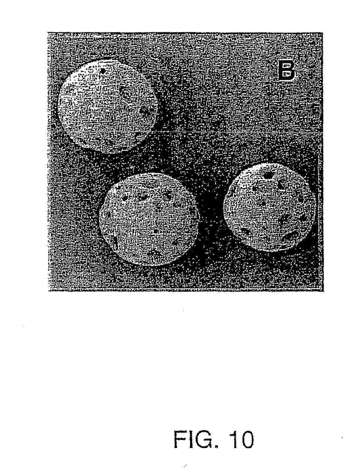

In an embodiment, a particle having macropores may be formed of agarose. A depiction of such a particle is shown in FIG. 10. A particle may be in the form of a spherical bead. The particle may include a plurality of macropores on its surface and interior.

In an embodiment, agarose may be used as a starting material for a macroporous particle because it is biocompatible and may be capable of interacting with biomolecules and living organisms. Activated agarose may demonstrate an affinity interaction with bacteria and microorganisms. To facilitate this interaction, specific properties on particles may be used to target specific microorganisms or cells. Processed agarose, in which sulfate groups have been eliminated from the agarose chain, may consist of an uncharged hydrophilic matrix with primary and secondary alcohols that can be used for activation and attachment. For example, the chemical surface of particles may be modified by oxidizing adjacent diols into aldehyde groups. Using sodium meta-periodate (NaIO₄) aliphatic aldehydes may be obtained that can be used in reductive amination coupling procedures.

In an embodiment, macroporous particles may be formed by suspension polymerization using a gel. Size, shape, and uniformity of the particle may depend on the hydrophilic or hydrophobic additives used to stabilize the emulsion. Pore size may be determined by agarose concentration of the gel. Mechanical properties, such as gel strength, may be affected by the molecular weight of the agarose. In one embodiment, suspension polymerization may be accomplished using a biphasic system containing the agarose monomer and emulsion stabilizers. A dispersion of a hydrophilic emulsifier (such as TWBBN 85) in cyclohexane may be added to a stirring aqueous solution of agarose at 60°C for 5 min to produce an oil-in-water emulsion. Fine particles of agarose stabilized by the emulsifier may be formed in this step. Next, a solution of a hydrophobic emulsifier (such as SPAN 85) may be added to the first emulsion forming a water-in-oil emulsion. Afterwards, the water-in-emulsion may be cooled to room temperature. Polymeric particles may appear at about 40°C. The aggregation of droplets, which may be referred to as flocculation, may form a matrix with oil droplets that will form pores or conduits in the beads. The particles may be washed with distilled water and alcohol, sized with industrial sieves, and preserved in water.

Emulsifiers added to the hydrophilic and/or hydrophilic phases and the concentration of the agarose solution may influence the quality of the beads. Additionally, mixing speed, nature of the agitation, and temperature during the preparation process may be important. The stability of the solutions may depend on the selected emulsifiers and the solvents used.

A particle may be of a substantially spherical shape. Particles with spherical geometry may enhance the available area for surface interaction with the analytes. Creating pores within the particles may also increase surface area. Particles may have large connecting flow pores in addition to normal diffusion pores. A macroporous particle may have improved mass transfer properties compared to a non-macroporous particle.

A particle may have a diameter of between about 250-300 microns. Macropores in a particle may be less than about 1 micron. Different pore sizes and shapes may allow for the entrapment and detection of a variety of analytes, including, but not limited to, cells, bacteria, DNA oligomers, proteins/antibodies, and small molecules.

An alternative process to suspension polymerization may be the use of a foaming agent to vary the porosity of the particles. For example, the decomposition of azides or carbonates during polymerization may allow incorporation of nitrogen or carbon dioxide "bubbles" into the particles. Because the gelling point for agarose is 40°C, the decomposition reaction should be performed at low temperatures.

Another alternative to suspension polymerization may be the use of molecular imprinting. The imprinting of particles may occur by non-covalent and covalent methods. Non-covalent imprinting may be based on non-covalent interactions such hydrogen bonds, ionic bonds, and Van der Waals forces between functional monomer and a temmember. The stability of the monomer-temmember complex prior to polymerization may depend on the affinity constants between the temmember and the functional monomers. In the covalent method, the bonds formed between the functional monomer and the temmember may be cleaved once the polymerized matrix is obtained.

Within the covalent and non-covalent based approaches, there may be different methods for making molecular imprinted polymers. One approach may involve grinding the imprinted polymer to reduce their size to approximately 25 µm and expose the imprinted surfaces. Another technique, which may be referred to as 'surface temmember polymerization,' uses water and oil. In this technique, the water-soluble temmember may interact with the functional monomer at the water-oil interface. The complex monomer-temmember in the organic phase may be polymerized yielding a polymer-imprinted surface. This technique may allow the imprinting of water-soluble substances like zinc ions.

Other methodologies for imprinting polymers may be suitable. Molecular imprinting on microgel spheres may be a convenient procedure for imprinting agarose because the imprinted gel does not need to be reduced in size by grinding as in conventional molecular imprinting. Discrete imprinted microgels and imprinted microspheres may be synthesized by cross-linking polymerization of the monomer in the presence of the temmember, a process known as "precipitation polymerization."

Surface temmember polymerization and precipitation polymerization may be suitable alternative techniques to chemical surface modification of regular particles. Both techniques may be suitable for printing agarose with such temmembers as bacterial spores. A chromatography column mounted with imprinted beads may be a fast method for evaluating the efficacy of the imprinted beads. For example, bacteria may be re-bound, exposed to the fluorescent calcium-sensitive indicator known as calcein, and detected by fluorescence spectroscopy.

Molecular imprinting may allow the exploitation of organisms as reactors. The pores in the particle may facilitate feeding of entrapped microorganism reactants and cause them to produce a desired product. Molecular imprinting may be used for encapsulating bacteria such as the Rhizobium organisms into agarose. These bacteria may convert nitrogen from the atmosphere into ammonia. By "feeding" these bacteria nitrogen, ammonia may be produced. The pores encapsulating the bacteria may retain an imprint of the organism for morphologic studies of the bacteria's surface.

High-performance liquid chromatography and fluorescent assays may be a valuable tool for studying the molecularly imprinted polymers. The fluorescent dye acridine orange may stain agarose beads so they may be morphologically analyzed with confocal scanning laser microscopy. Other morphological studies include atomic force microscopy, scanning electron microscopy, and microtome techniques. Characterization of the surface area of the beads, may be achieved by measuring the adsorption isotherm and using the Brunauer, Emmet, and Teller equation.

In some embodiments, the surface of a particle may be chemically modified. In other embodiments, chemical functionality, including, but not limited to non-specific (i.e., functional groups) and highly specific (i.e., bio-ligands such as antibodies) may be localized into the confines of the pore region. Chemical functionality may facilitate the entrapment of a variety of analytes.

In an embodiment, a particle may include a receptor that includes a particular metal. The metal may be capable of binding a material that is characteristic of a particular analyte. For example, a particle may be formed that includes terbium (III). Terbium (III) forms a luminescent complex with dipicolinic acid, a substance unique to spores.

### Example:

Macroporous beads were prepared using the method for biphasic suspension polymerization method described herein. The beads so obtained were analyzed using light and fluorescence microscopy. The transparency of the agarose beads permitted the visualization of the fluorescent beads in different sections of the agarose beads. The presence of pores was confirmed by adding 1 µm fluorescent beads. Using light and fluorescence microscopy, the presence of conduits could not be conclusively determined. The beads accumulated into voids present in the bead, probably the ends of conduits.

Experiments were initially performed using Merck's Omnipure agarose powder. Low yields of non-spherical particles ranging between 250 and 300 µm were obtained. Experiments performed with an exaggerated amount of the hydrophilic emulsifier, 3.5 mL span 85 resulted in beads without pores but with a rough surface. By reducing the amount of the hydrophobic emulsifier, massive gellation due to the poor stabilization of the agarose particles in the oil in water emulsion occurred.
Agarose aqueous solution concentration 4% (w/v), o/w emulsion: 0.7mL tween 80/10 mL cyclohexane w/o emulsion: 7 mL span 85/75 mL cyclohexane

**Table 1. Effect of the stirring speed on the fabrication of porous agarose bead**

| Stirring speed with a magnetic stirrer | Fluorescence and light microscopy | Apparent porosity | Efficiency Size 250-300 µm |
|---|---|---|---|
| 10 | With oil inclusions, regular integrity | A few | Less than 10% |
| 9 | Medium integrity | None | About 10% |
| 8 | Better integrity | A few but more than stir at 10 | About 10% |

The effect of stirring speed has been briefly evaluated. With higher stirring speeds the integrity of the beads was poor. Smaller particles are expected to be the result of faster stirring speeds, but exposure to higher physical stress only results in the disintegration of the beads. Trials performed under the same conditions using Sigma agarose gave similar results to Merck agarose, but with slightly higher yields around 20%. The integrity of the beads improved slightly suggesting better mechanical properties such as gel strength.

Experiments for producing homogeneous particles were performed using agarose obtained from Merck at a constant concentration of agarose solution and stirring. The results are shown in table 2.
Agarose aqueous solution concentration 4% (w/v), o/w emulsion: 0.7mL, tween 80/10 mL cyclohexane w/o emulsion: 7 mL span 85/75 mL cyclohexane

**Table 2. Effect of the emulsifier on the fabrication of homogeneous agarose beads**

| **Stirring speed with a magnetic stirrer** | **Fluorescence and light microscopy** | **Efficiency Size 250-300 µm** |
|---|---|---|
| **10** | Opaque beads | **About 10%** |
| **10** | Regular integrity | **About 10%** |
| **10** | **Bad integrity** | **Less than 10%** |

Excessive stabilization of the water in oil emulsion causes reduced flocculation and increases the formation of fines resulting in a lower yield. Performing the same experiment with a fixed stirrer speed of 8 (Coming stiffer/hot member, model # PC-420) slightly increased the yield. Magnetic stirring may not be appropriate for viscous solutions or the foam obtained during emulsification (creaming).

### Bead Selection Techniques

Sensor arrays that use beads (either non-porous or porous) can be used to determine the presence of a variety of analytes. Typically, the beads include a receptor that binds to an analyte. The receptor may also bind to an indicator. The indicator typically produces a signal in the presence of an analyte that is different from a signal produced in the absence of an analyte. The selection of beads for use with a particular analyte may be important to the success of the sensor array. In general, a bead should have a high affinity for the analyte and produce an easily detectable signal. A method is described herein which may be used to determine an optimal receptor for a given analyte and indicator.

One method used to determine the presence of an analyte is a displacement assay. In a displacement assay a bead that includes a receptor is preloaded with an indicator. The indicator interacts (e.g., is bound to) the receptor such that the bead appears to have a specific color or fluorescence due to the indicator. When a solution that includes an analyte is brought into contact with the bead, the analyte may displace the indicator from the receptor. This displacement may cause a loss of color or fluorescence of the bead since the indicator is no longer associated with the bead. By measuring the loss of color or fluorescence of the bead, the presence of an analyte may be determined. The success of such an assay for determining the presence of an analyte is dependent, in part, on the interaction of the receptor with the analyte and the indicator. Generally, the bead should show a maximum color and fluorescence when an indicator is bound to the receptor, however, the indicator should be easily displaced by the analyte.

In one embodiment, a plurality of beads having a variety of receptors may be produced. In one embodiment, the receptors may be formed from a variety of different receptor types. Alternatively, the beads may have similar receptors. For example, techniques are well known to create libraries of peptide, peptide mimics, or nucleotides upon polymeric beads. For peptide libraries up to 20ⁿ different beads may be produced in a library, where n is the number of amino acids in the peptide chain. Nucleic acid libraries may have up to 4ⁿ different beads where n is the number of nucleic acid bases. Because of the large number of different beads in these libraries, the testing of each individual bead is very difficult.

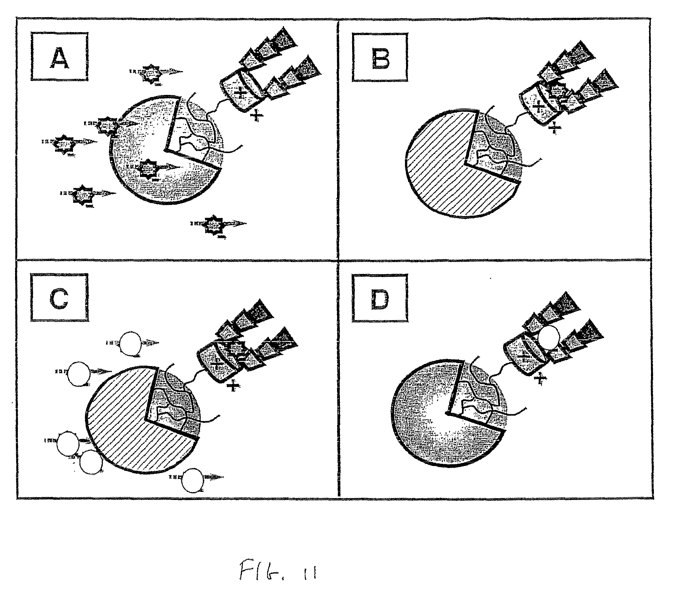

FIG. 11 depicts a schematic drawing of a method for optimizing a receptor on a bead. In FIG 11A, a bead is depicted that includes a receptor X. Receptor X is composed of 6 subparts that extend from a base. The base is coupled to the bead. The bead is first contacted with an indicator, denoted as the stars in FIG. 11A. The indicator interacts with each of the beads in the library, binding to the receptors. FIG. 11B shows the indicator coupled to the receptor of the bead. As depicted in FIG. 11b, the color or fluorescence of the bead is altered due to the interaction of the indicator with the receptor. The change in color or fluorescence of the bead indicates that the bead is capable of interacting with the indicator.

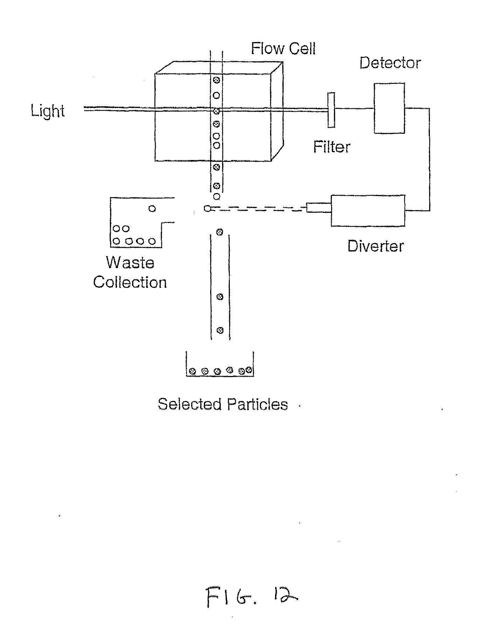

When a plurality of beads is used, the indicator will bind to the beads at various strengths. The strength of binding is typically associated with the degree of color or fluorescence produced by the bead. A bead that exhibits a strong color or fluorescence in the presence of the indicator has a receptor that binds with the indicator. A bead that exhibits a weak or no color or fluorescence has a receptor that only weakly binds the indicator. Ideally, the beads which have the best binding with the indicator should be selected for use over beads that have weak or no binding with the indicator. FIG. 12 depicts a schematic of a flow cytometer which may be used to separate beads based on the intensity of color or fluorescence of the bead. Generally, a flow cytometer allows analysis of each individual bead. The beads may be passed through a flow cell that allows the intensity of color or fluorescence of the bead to be measured. Depending on the measured intensity, the bead may be collected or sent to a waste collection vessel, as indicated in FIG. 12. For the determination of an optimal bead for interaction with an indicator, the flow cytometer may be set up to accept only beads having an color or fluorescence above a certain threshold. Beads that do not meet the selected threshold, (ie., beads that have weak or no binding with the indicator) are not collected and removed from the screening process. Flow cytometers are commercially available from a number of sources.

After the bead library has been optimized for the indicator, the beads that have been collected represent a reduced population of the originally produced beads. If the population of beads is too large, additional screening may be done by raising the intensity threshold. Now that the beads that exhibit optimal interaction with a receptor have been identified, the remaining beads are optimized for displacement of the indicator by the analyte of interest. Thus, the remaining beads are treated with a fluid that includes the analyte of interested, as depicted in FIG. 11C. The analyte is represented by the circle. For some beads, the analyte will cause displacement of the indicator, causing the color or fluorescence of the bead to be reduced, as depicted in FIG. 11D. The intensity of the color or fluorescence of the bead after it interacts with an analyte will be based on how the competitive displacement of the indicator. A bead that exhibits weak or no color or fluorescence when treated with an analyte is the most desirable. Such beads show that the analyte is readily bound by the receptor and can readily displace the indicator from the receptor.

Once again a flow cytometer may be used to determine the optimal beads for use in an assay. A library of beads that have been optimized for interaction with an indicator are treated with a fluid that includes an analyte. The treated beads are passed through a flow cytometer and the beads are separated based on intensity of color or fluorescence. The beads that exhibit a color or fluorescence below a predetermined intensity are collected, while beads that show a color or fluorescence above the predetermined intensity are sent to a waste collection. The collected beads represent the optimal beads for use with the selected analyte and indicator. The identity of the receptor coupled to the bead may be determined using known techniques. After the receptor is identified, the bead may be reproduced and used for analysis of samples.

### HIV detection

More than 35 million HIV-infected people live in developing countries with significant resource limitations. Although effective antiretroviral therapy has been available in developed countries for almost a decade, fewer than 300,000 people living in developing countries are believed to be receiving treatment. One major obstacle, the cost of antiretroviral medications (ARVs), is being addressed by price reductions and the advent of generic versions of ARVs. A second obstacle, the cost and technical requirements of the sophisticated laboratory tests used to initiate and monitor HIV treatment, remains to be addressed.

Of particular importance, measurements of CD4+ T lymphocytes are essential for evaluating HN-infected patients. CD4 counts, expressed as either absolute numbers of CD4 cells per milliliter of blood, a percentage of total T lymphocytes, or as the ratio of CD4:CD8 T lymphocytes, have enormous prognostic and therapeutic implications, and form the basis for most treatment decisions. In developed countries, CD4 counts are performed using flow cytometers, which use lasers to excite fluorescent antibody probes specific for CD4 and other T cell markers. The emitted light is collected by a series of photomultiplier tubes, and these signals are analyzed to differentiate CD4+ T cells from CD8+ T cells and other cellular components of blood. Costs for flow cytometers range from $30,000 to $100,000 per machine. This expense, as well as their need for high wattage electricity, regular maintenance and costly reagents, makes flow cytometers impractical, and unsustainable in resource-scarce settings.

Several preliminary efforts have been made to develop alternative, affordable CD4 counting methods for resource-poor settings. Single-purpose flow cytometers for CD4 counting have been developed, but remain costly. A second approach has been to use low-cost immunomagnetic separation of CD4 cells from other blood cells, followed by standard cell counting methods using a light microscope. While significantly cheaper, these methods are low throughput and less accurate than the flow cytometry-based methods.

To address the significant need for low-cost CD4 counts for resource-poor settings, we have applied recent advances in microdetection technologies to the development of accurate, affordable and portable CD4 counts. Deecribed herein is an affordable microchip-based CD4 assay, including basic performance characteristics of a prototype version and preliminary evaluations in HIV-infected and control subjects.

A microporous lymphocyte capture membrane was used in a membrane based flow sensor as previously described. For preliminary studies, CD4 cells were purified by immunomagnetic separation from buffy coats obtained from healthy donors. All CD4 preparations were greater than 98% pure by flow cytometric analysis. CD4 cells labeled with Alexa488-conjugated anti-CD4 antibodies were introduced to the flow cell in amounts ranging from 0 to 200,000 cells in 1 ml of cell media, and washed with 2 to 5 milliliters of phosphate-buffered saline (PBS).

For studies on human subjects, 20 microliters of whole blood obtained by venipuncture were incubated with 2 microliters of Alexa488- or Alexa647-conjugated antibodies to CD3, CD4 and/or CD8, After 8 minutes, the sample was introduced directly to the flow chamber without further processing, and then washed with 2 milliliters of PBS. Images of stained cells captured in the microchamber were then obtained and processed for analysis. For some experiments, after image collection 2% glutaraldehyde was introduced into the flow cell and the cells fixed for scanning electron microscopy.

The membrane based flow sensor was immobilized on the stage of a microscope system equipped with a medium-pressure mercury lamp as a light source. For each study subject, a total of five non-overlapping regions of the lymphocyte capture membrane in the floor of the microchamber were imaged using a digital camera (DVC, Austin, Texas) attached to the workstation. Each region was imaged twice, once under a red wavelength absorption filter (for detection of Alexa-488 fluorescence) and once under a green wavelength absorption filter (for detection of Alexa-647 fluorescence). Except for the preliminary studies, these two images were merged to produce a single image prior to analysis.

Each image was analyzed using a custom algorithm developed in a commercial image processing software package (Image-Pro Plus). Thresholds for red, green and blue intensity were established for optimal definition of lymphocytes against the background, and lymphocytes were characterized by their geometry (size and shape). Cells thus identified were then counted in an automated fashion, with results recorded in a spreadsheet as numbers of CD4CD3-, CD4+CD3+, CD8+CD3+, and CD8+CD3- and CD4+CD8+ cells, depending on the combination of antibodies used.

Blood was obtained by venipuncture from healthy volunteers or HIV-infected subjects at the Massachusetts General Hospital. Samples were processed in parallel by flow cytometry at either the MGH clinical laboratories or at the research facility, using standard 4-color protocols on a FACScalibur flow cytometer. The study was approved by the Institutional Review Board at the Massachusetts General Hospital, and informed consent was obtained from all study subjects.

Results obtained by the automated microchip method were compared directly with results obtained by flow cytometry for each of the study subjects, and correlated using a standard statistical software package.

Previously, we developed microchip immunoassays using antibody-coupled microbeads immobilized in an inverted pyramidal microchamber. This design has proven extremely effective as a microELISA platform for antigen and antibody detection. For CD4 counting, flow chambers were re-engineered with a floor that consisted of a porous plastic grid, upon which rests a disposable lymphocyte capture membrane filter. These filters have a predetermined pore size ranging from 0.2 to 30 microns. In preliminary studies, pore sizes of between 2 and 5 microns proved optimal for microfluidics of the flow chamber, and for retention of lymphocytes. The unwanted red blood cells and platelets pass through the pores and out of the flow chamber prior to imaging, significantly reducing background fluorescence and improving image quality.

To determine the correlation between fluorescence light intensity detected in our system and the number of labeled CD4 cells, we added an increasing number of purified CD4+ lymphocytes in 1 mL of RPMI to individual microchambers and measured detectable fluorescence. Individual CCD4+ T cells are discernible in the dilute samples. Importantly, there is a linear correlation between the number of cells in the sample and the light intensity when measured from a digital image using a pixel analysis. This established proof of principle that the microchamber and image analysis system could be used to accurately measure and detect populations of lymphocytes labeled with fluorescent markers.

We next developed an assay to measure CD4 cells directly from whole blood, in a single-step no-lyse system. Using blood obtained by venipuncture from healthy control and HIV-infected subjects, 20 microliters of whole blood were incubated with 2 microliters of fluorescently labeled antibodies to CD3, CD4 or CD8. Based on initial studies of photobleaching and signal intensity, we used only the Alexa line of fluorophores (Molecular Probes) in studies on human subjects. The availability of only Alexa-488 and Alexa-647 conjugated antibodies against CD antigens limited us to two-color imaging. After an 8 minute incubation, samples were diluted in 480 microliters of PBS, introduced to the flow chamber, washed with 2 mL PBS, and imaged. The total time from blood processing to image analysis was under 15 minutes.

Alexa488-conjugated antibodies label the CD4 cells green, and Alexa647-conjugated antibodies to CD3 label all T lymphocytes red. Automatic merging of the images allows the distinction between the CD3+CD4+ T lymphocytes of interest, which appear yellow, the CD4+CD3- monocytes (green), and CD3+CD4- T cells (red). For each study subject, five non-overlapping images are obtained, increasing the size of the sample cell population and improving accuracy.

Scanning electron micrography of a typical sample prepared as described above and processed inside the microchamber. Unwanted red blood cells are not retained on the filter, but pass through the pores with the saline wash, making lysing unnecessary. This, combined with the high volume wash (100-fold excess for the 20 microliter sample) significantly reduces any background fluorescence and improves the image quality.

Results for CD4 percentages (number of CD4+CD3+ cells/number of total CD3+ cells) and CD4:CD8 ratios (CD4 percentage/CD8 percentage) obtained from the microchip system were compared with results obtained by flow cytometry for each of the study subjects. Comparisons of the microchip method with flow cytometry for CD4:CD8 ratios gave good correlations.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## Claims

1. An analyte detection device comprising:
a body;
a porous membrane coupled to the body;
a top member positioned at a spaced distance above the porous membrane such that a first cavity is formed between the top member and the porous membrane, wherein the top member covers at least a portion of the porous membrane, and wherein the top member is substantially transparent to light; and
a bottom member positioned below the porous membrane, wherein the bottom member is configured to receive fluid flowing through the porous membrane during use.

2. The device of claim 1, wherein the porous membrane comprises pores having a diameter between about 0.2 microns to about 12 microns.

3. The device of claim 1, wherein the top member is substantially transparent to visible light.

4. The device of claim 1, wherein the bottom member is substantially transparent to visible light.

5. The device of claim 1, wherein the top member is substantially transparent to visible light, and wherein the bottom member is substantially transparent to visible light.

6. The device of claim 1, wherein the top member is substantially transparent to ultraviolet light.

7. The of claim 1, wherein the bottom member is substantially transparent to ultraviolet light.

8. The device of claim 1, wherein the top member is substantially transparent to ultraviolet light, and wherein the bottom member is substantially transparent to ultraviolet light.

9. The device of claim 1, wherein the bottom member comprises an indentation configured to receive the membrane.

10. The device of claim 1, wherein the bottom member comprises a first indentation configured to receive the filter and a second indentation which is configured to receive fluid passing through the membrane during use.

11. The device of claim 1, further comprising a membrane support coupled to the membrane, wherein the membrane support is composed of a porous material.

12. The device of claim 11, wherein the membrane support comprises pores that allow fluid to flow through the membrane support at a speed that is equal to or greater than the speed that fluid passes through membrane.

13. The device of claim 11, wherein the membrane support provides sufficient support of the membrane during use to inhibit sagging of the membrane.

14. The device of claim 11, wherein the membrane support is configured to maintain the membrane in a substantially planar orientation during use.

15. The device of claim 1, further comprising a gasket positioned between the membrane and the top member.

16. The device of claim 1, wherein the top member comprises a fluid inlet configured to allow fluid to be introduced to the membrane through the top member.

17. The device of claim 1, wherein the bottom member comprises a fluid outlet configured to allow fluid to pass from the membrane out of the analyte detection device.

18. The device of claim 1, wherein the top member comprises a fluid inlet configured to allow fluid to be introduced to the membrane through the top member, and wherein the top member comprises a wash fluid outlet configured to allow fluid to pas from the membrane out of the analyte detection device during a washing operation.

19. The device of claim 1, further comprising a body and a cap, wherein the top member, the membrane and the bottom member are disposed within the body, and wherein the cap secures the top member, the membrane and the bottom member within the body.

20. The device of claim 1, wherein top member is composed of an acrylate polymer.

21. The device of claim 1, wherein the top member and the bottom member are composed of an acrylate polymer.

22. An analyte detection system comprising:
an analyte detection device, the analyte detection device comprising:
a body;
a porous membrane coupled to the body;
a top member positioned at a spaced distance above the porous membrane such that a first cavity is formed between the top member and the porous membrane, wherein the top
member covers at least a portion of the porous membrane, and wherein the top member is
substantially transparent to light; and
a bottom member positioned below the porous membrane, wherein the bottom member is configured to receive fluid flowing through the porous membrane during use
a detector optically coupled to the porous membrane, wherein the detector is configured to view at least a portion of the membrane through the window;
a fluid delivery system coupled to the analyte detection device, wherein the fluid delivery system is configured to deliver fluid samples to the analyte detection device.

23. The system of claim 22, wherein the detector comprises a CCD camera.

24. The system of claim 22, wherein the fluid delivery system comprise one or more pumps.

25. The system of claim 22, wherein the fluid delivery system comprises a plurality of pumps, each of the pumps coupled to a different fluid storage container.

26. The system of claim 22, wherein the fluid delivery system comprises one or pumps and one or more filters, wherein the filters are configured to filter fluids before the fluids are delivered to the analyte detection device.

27. The system of claim 22, wherein the analyte detection device comprises a fluid inlet, and wherein the fluid delivery system comprises a plurality of pumps each of the pumps coupled to a different fluid storage container and a manifold, wherein the manifold is configured to redirect fluid from received from at least a portion of the pumps to the fluid inlet.

28. The system of claim 22, wherein the detector comprises a microscope.

29. The system of claim 22, further comprising a programmable controller coupled to the fluid delivery system.

30. The system of claim 29, wherein the programmable controller is further coupled to the detector.

31. A method of sensing an analyte in a fluid comprising:
passing the fluid across a porous membrane configured to capture the analyte on the porous membrane;
detecting an image of matter captured on the porous membrane; and
determining if the analyte is present on the porous membrane.

32. The method of claim 31, further comprising passing the analyte to a sensor array if the image meets the user-defined criteria.

33. The method of claim 32, wherein the sensor array comprises a porous particle.

34. The method of claim 31, wherein determining if the analyte is present comprises comparing the shape of the matter to user-defined criteria.

35. The method of claim 31, determining if the analyte is present comprises comparing the size of the matter to user-defined criteria.

36. The method of claim 31, determining if the analyte is present comprises comparing the aggregate area of the matter to user-defined criteria.

37. The method of claim 31, determining if the analyte is present comprises comparing the color of the matter to user-defined criteria.

38. The method of claim 31, determining if the analyte is present comprises comparing the fluorescence of the matter to user-defined criteria.

39. The method of claim 31, determining if the analyte is present comprises comparing the fluorescent intensity of the matter to user-defined criteria.

40. The method of claim 31, further comprising applying a stain to the matter captured on the membrane.

41. The method of claim 31, further comprising collecting a sample of an analyte in a fluid using an air collection device.

42. The method of claim 31, further comprising passing a background fluid through the filter and detecting an image captured on the porous membrane prior to passing the fluid containing the analyte across the porous membrane.

43. The method of claim 31, further comprising performing a lateral flush to clean the surface of the membrane prior to passing the fluid containing the analyte across the membrane.

44. The method of claim 31, further comprising performing a back flush to clean the surface of the membrane prior to passing the fluid containing the analyte across the membrane.

45. The method of claim 31, wherein detecting an image is performed using a CCD detector.

46. The method of claim 31, wherein detecting an image is performed using a microscope.

47. The method of claim 31, further comprising further comprising passing a visualization agent across the membrane after the fluid is passed over the membrane.

48. The method of claim 31, further comprising performing a lateral wash of the membrane after detecting an image.

49. The method of claim 31, further comprising performing a back wash of the membrane after detecting an image.

50. A method of analyzing an analyte collected on a membrane comprising:
passing a fluid sample across a membrane, wherein the fluid sample comprises an analyte that is at least partially retained by the membrane;
adding a visualization agent to material collected on the membrane when the fluid sample is passed across the membrane;
collecting an image of the collected material using white light, at a first wavelength of light, a second wavelength of light, and a third wavelength of light, wherein the analyte comprises a color corresponding to the first wavelength of light;
forming a first mask corresponding to an image of the collected material at the second wavelength of light;
forming a second mask corresponding to an image of the collected material at the third wavelength of light;
subtracting the first mask and the second mask from the image of the collected material in white light.

51. The method of claim 50, wherein the wavelengths of light are selected from the group consisting of red, blue and green.

52. The method of claim 50, wherein the collecting the image data and forming the masks is performed by a computer.

53. The method of claim 50, further comprising determining the amount of analyte present on the membrane by analysis of the image resulting from subtracting the first mask and the second mask from the image of the collected material in white light.

54. The method of claim 50, wherein the images are collected using a CCD detector.

55. The method of claim 50, wherein the images are collected using a CCD detector coupled to a microscope.

56. A particle for detecting an analyte in a fluid comprising a receptor coupled to a polymeric resin, wherein the polymeric resin comprises a plurality of pores having a diameter of less than about 1 µm.

57. The particle of claim 56, further comprising a receptor coupled to the surface of one of the pores.

58. The particle of claim 56, wherein the particle comprises agarose.

59. The particle of claim 56, wherein the particle is substantially spherical.

60. The particle of claim 56, wherein the particle has a a diameter of between about 100 to 500 microns.

61. The particle of claim 56, wherein the particle is configured to entrap microbes.

62. A method for forming a porous particle, comprising:
forming an emulsion of a polymeric resin in an aqueous solution;
reducing the temperature of the emulsion to produce the porous particle, wherein the porous particle comprises a plurality of pores having a diameter of less than about 1 µm.

63. The method of claim 62, wherein forming an emulsion comprises adding an emulsifier to a mixture of the polymeric resin in water.

64. A porous particle, formed by the method of claim 62.

65. A method for detecting a microbe, comprising:
passing the fluid over a porous particle configured to capture the microbe; and
detecting the microbe with a detector.

66. The method of claim 65, wherein a receptor configured to receive the microbe is coupled to the porous particle.

67. A system for detecting an analyte in a fluid comprising:
a light source;
a sensor array, the sensor array comprising a supporting member comprising at least one cavity formed within the supporting member;
a particle, the particle positioned within the cavity, wherein the particle is configured to produce a signal when the particle interacts with the analyte during use, and wherein the particle comprises a receptor coupled to a polymeric resin, wherein the polymeric resin comprises a plurality of pores having a diameter of less than about 1 µm; and
a detector, the detector being configured to detect the signal produced by the interaction of the analyte with the particle during use;
wherein the light source and detector are positioned such that light passes from the light source, to the particle, and onto the detector during use.

68. A method of sensing an analyte in a fluid comprising:
passing a fluid over a sensor array, the sensor array comprising at least one particle positioned within a cavity of a supporting member, wherein the particle comprises a receptor coupled to a polymeric resin, wherein the polymeric resin comprises a plurality of pores having a diameter of less than about 1 µm;
monitoring a spectroscopic change of the particle as the fluid is passed over the sensor array, wherein the spectroscopic change is caused by the interaction of the analyte with the particle.

69. A method of sensing an analyte in a fluid comprising:
passing the fluid across a porous membrane configured to capture the analyte on the porous membrane;
applying a visualization agent to the particles on the porous membrane;
detecting an image of matter captured on the porous membrane with a detector at a plurality of wavelengths of light;
detecting an image of matter captured on the porous membrane at a specific wavelength pf light, wherein the specific wavelength of light represents light that is not indicative of the presence of the analyte.

70. A method of forming a sensor array comprising:
forming a plurality of cavities in a supporting member;
forming a plurality of particles, wherein each particle comprises a receptor coupled to a polymeric resin, wherein a plurality of different receptors are coupled to the particles;
interacting the plurality of particles with an analyte;
determining which particles interact with the analyte and the extent to which the interact with the analyte;
separating particles that interact with the analyte and meet a predetermined criteria from particles that do not substantially interact with the analyte or do not meet a predetermined criteria;
adding the separated particles that interact with the analyte and meet the predetermined criteria to a sensor array.

71. The method of claim 70, wherein separating the particles comprises separating the particles using a flow cytometer.

72. A method of sensing an HIV virus in blood comprising:
passing blood across a porous membrane, wherein the porous membrane is configured to retain white blood cells while allowing other materials in the blood sample to pass through; and
determining the amount of white blood cells and the type of white blood cells captured by the membrane.

73. An analyte detection device comprising:
a body;
a porous membrane coupled to the body.
